# EUROPEAN PATENT SPECIFICATION

(11) **EP 3 713 500 B1**
(45) Date of publication and mention of the grant of the patent: **03.09.2025**
(21) Application number: 18881842.1
(22) Date of filing: 23.11.2018
(51) Int. Cl.: A61B 10/02, A61B 10/04, A61M 25/04

(54) **BALLOON-ANCHORED BIOPSY DEVICE**
BALLONVERANKERTE BIOPSIEVORRICHTUNG
DISPOSITIF DE BIOPSIE ANCRÉ PAR BALLONNET

(30) Priority: 24.11.2017 SG 10201709763Q
(43) Date of publication of application: 30.09.2020
(73) Proprietor: National University Hospital (Singapore) Pte Ltd, Singapore 119228 (SG); National University of Singapore, Singapore 119077 (SG); Irnovate Pte Ltd, Singapore 138638 (SG)
(72) Inventor: MANGAT, Kamarjit Singh, Edgbaston, Birmingham B17 0HY (GB); HONG, Rachel Tsui Ying, Singapore 640410 (SG); LAU, Andrew Chin Cheung, Singapore 750472 (SG); TAN, Gabriel Hong Chun, Singapore 558844 (SG); WIGHT, Ronald Craig, Singapore 659443 (SG)
(74) Representative: IXAS Conseil
(86) International application number: PCT/SG2018/050576
(87) International publication number: WO 2019/103694

(56) References cited:
- WO-A1-2014/141267
- WO-A1-2015/153931
- WO-A1-2016/115450
- CN-A- 106 725 249
- US-A- 4 513 754
- US-A- 4 907 598
- US-A- 5 827 305
- US-A1- 2005 288 550
- US-A1- 2007 249 940
- US-A1- 2010 114 031
- US-A1- 2012 053 485
- US-A1- 2014 194 776
- US-A1- 2015 032 129
- US-A1- 2015 141 836
- US-A1- 2015 141 836
- US-A1- 2016 038 127
- US-A1- 2017 079 519
- US-B2- 9 757 099

## Description

### FIELD OF THE INVENTION

The present invention relates to biopsy devices, and more particularly to a balloon-anchored biopsy device for acquiring a biopsy sample of a target organ.

### BACKGROUND OF THE INVENTION

Current methods for performing liver biopsies may include an inherent risk of severe complications, which may often result in patients opting to delay the biopsy procedure, thus delaying subsequent diagnosis and intervention of liver dysfunction. The methods may include open surgery, percutaneous liver biopsy (PLB), and transjugular liver biopsy (TJLB).

Open surgery liver biopsy is the direct removal of liver tissue during a laparoscopic, or surgical procedure. Open surgical liver biopsy in modern practice may be utilized when there is already a surgical procedure underway.

Percutaneous liver biopsy (PLB) may involve extracting a core sample of liver tissue using a biopsy needle inserted through the abdominal wall. In PLB, the liver capsule is punctured, and a high penetration depth is needed to reach the parenchyma. This procedure may often provide good biopsy samples, but the procedure is invasive, painful, and may carry a risk of significant complications, including a risk of death (1 in 2500). If the first biopsy fails and additional biopsy samples are needed, additional needle punctures further increasing the risk of complications. Thus, PLB patients may be kept under observation for several hours after the procedure to ensure that there is no bleeding into the peritoneal cavity due to the puncturing of the liver capsule or vessels.

Transjugular liver biopsy (TJLB) involves accessing the liver through the insertion of a stiff metal catheter into the right jugular vein, and navigated thru the right chamber of the heart and into the hepatic vein of the liver. A large bore needle directed down the catheter is used to core the liver tissue. Multiple samples are often needed for satisfactory analyses.

TJLB may avoid the risk of undetected bleeding into the peritoneum since any bleeding from the needle punctures as in PJB run back into the hepatic vein. TJLB involves navigating a stiff metal catheter through major organs and blood vessels. Thus, TJLB procedures may also result in significant complications such as hemorrhaging, arrhythmia vessel perforation, pneumothorax, or death. Although TJLB may be considered to be safer than PLB, TJLB does incur new risks of complications related to its jugular access site.

Furthermore, the use of soft tissue (e.g., liver, kidneys and pancreas) biopsy needles may be broadly classified into two biopsy methods: fine needle aspiration and core needle biopsy. Since the core needle biopsy method may preserve the native tissue structure, this method may be useful for the diagnosis of many soft tissue pathologies. Core needle biopsy may include, for example, the Tru-cut mechanism. Tru-cut needles systems are typically stiff and with lengths no longer than 70 cm. As a result, the stiffness and length of the Tru-cut needles preclude the possibility of navigating the Tru-cut needles into the liver via peripheral vascular access routes, that are generally considered to be safer with less risk to the patient. Hence, much effort been put into the design of flexible, long (>70 cm length) Tru-cut biopsy needles that could allow the use of peripheral access routes.

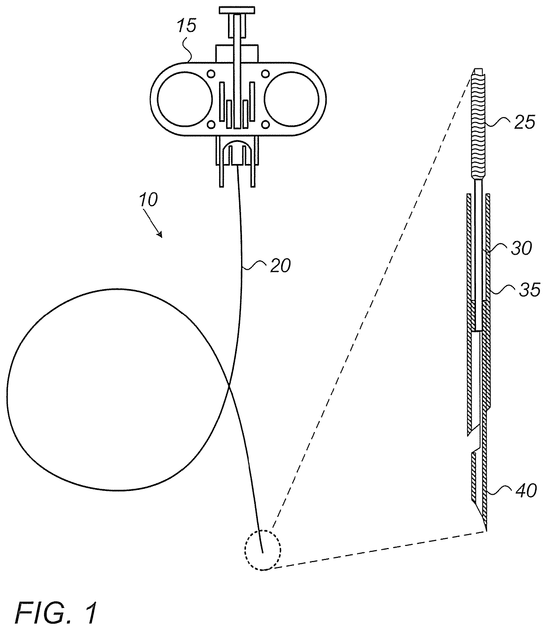

Fig. 1 schematically illustrates a long, flexible Tru-cut biopsy needle 10 as disclosed, for example, in U.S. Patent Nos. 5,273,051 and 4,907,598. A stylet 40 and a cutting cannula 35 at a distal end of a flexible sheath 20 of needle 10 are used to implement the Tru-cut mechanism. What has changed in migrating from a stiff Tru-cut needle to a flexible tru-cut needle, however, are the additions of long, flexible, co-axial cutting cannula tube 35 and a stylet wire 30 that respectively connect cutting cannula 35 and stylet 40 at the distal end of flexible Tru-cut biopsy needle 10 to a handle 15 at a proximal end of flexible Tru-cut biopsy needle 10, which includes with a spring-loaded firing mechanism. Stylet wire 30 may be pushed or pulled from the handle to control the stylet. Firing of the spring-loaded mechanism in handle 15 pushes cutting cannula tube 25 forward, which subsequently pushes cutting cannula 35 forward to shear through the soft tissue of the liver and collect to the soft tissue sample for the biopsy.

No such peripheral access (e.g., transcephalic) possibilities using core needle biopsy have been realized since flexible Tru-cut biopsy needles 10 have the following problems: Firstly, flexible Tru-cut biopsy needle 10 exhibit diminished force transfer along a length of flexible sheath 20 in that the flexible components dampen the propagation of force from the spring-loaded firing mechanism in handle 15 at the proximal end, to cutting cannula 35 at the distal end of needle 10. This results in an insufficient force applied to cutting cannula 35 to cleanly shear the soft tissue to obtain viable-sized, tissue samples in terms of sample diameter and length.

Secondly, the flexibility and length of flexible Tru-cut biopsy needle 10 also lead to insufficient in vivo stabilizing of cutting cannula tube 25 in the blood vessels resulting in a loss of pushability of needle 10 and the inability to maintain the position of stylet 40 when the spring-loaded mechanism in handle 15 is fired so as to perform the shearing and/or cutting of the soft tissue sample. (This is analogous to a fireman holding on to the end of a water hose to maintain directionality of the steam of water, and wherein the failure to hold onto the end will result in the water hose flopping around).

Finally in long flexible Tru-cut biopsy needle 10, cutting cannula 35 and stylet 40 in a flexible configuration may lose their optimal rotational and longitudinal alignments to one another during the navigation of the distal end of catheter 20 to the target organ. This may then require a technically challenging realignment step under fluoroscopic and/or ultrasound guidance. This realignment step might also not be always accomplished, given the limited fluoroscopic precision in current visualization systems as well as the limited pushability and torquability of flexible cutting cannula tube 25 and stylet wire 30.

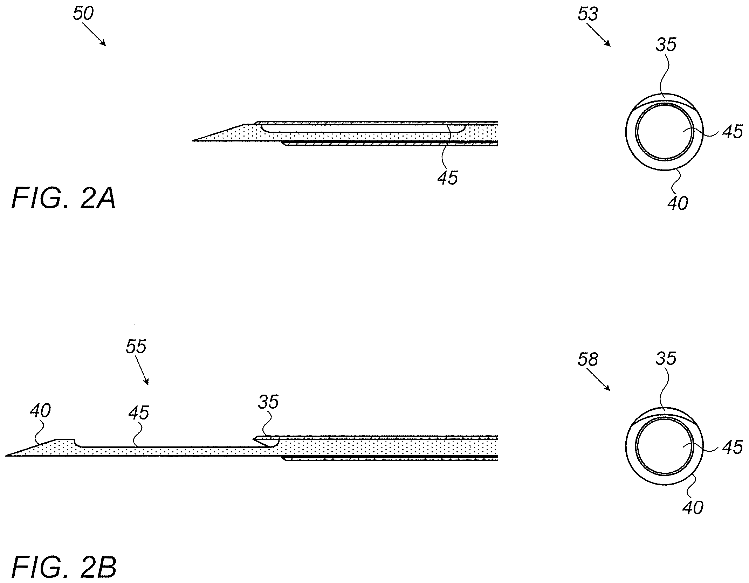

Fig. 2A schematically illustrates optimal alignments of cutting cannula 35 with stylet 40 of a non-flexible Tru-cut biopsy needle in a closed configuration 50. Fig. 2B schematically illustrates optimal alignments of cutting cannula 35 with stylet 40 of a non-flexible Tru-cut biopsy needle in an open configuration 55. The optimal rotational and longitudinal alignments of cutting cannula 35 and stylet 40 of the non-flexible Tru-cut needle's operation are shown in Figures 2A and 2B.

In closed configuration 50, a space between cutting cannula 35 and stylet 40 forms a specimen chamber 45. Cross-section 53 illustrates that cutting cannula 35 and stylet 40 are substantially opposite to one another in closed configuration 50. Stylet 40 is navigated through the venous system, for example, to the target region of the liver. The spring-loaded mechanism in handle 15 is then fired pushing stylet 40 into the soft liver tissue as shown in an open configuration 55. Cross-section 58 illustrates that cutting cannula 35 and stylet 40 still remain substantially opposite to one another in open configuration 55. When stylet 40 is retracted, cutting cannula 35 shears the soft tissue of the liver into specimen chamber 45.

In the case shown in Fig. 2, the alignment between cutting cannula 35 and stylet 40 are easily maintained since the cutting cannula and stylet are sufficiently stiff. Cutting cannula 35 and stylet 40 will not reversibly bend or twist to the point where their differing flexibilities lead to loss of alignment. Thus, the high pushability and torquability due to the stiffness of cutting cannula 35 and stylet 40 ensures that their alignment in the elements of the biopsy needle in the handle at the proximal end of the biopsy needle guarantees the alignment of cutting cannula 35 and stylet 40 at the distal end.

However, when cutting cannula tube and stylet wire are fabricated with the desired flexibility for peripheral access, the reverse occurs - the alignments are easily lost because both the cutting cannula tube and stylet wire are sufficiently flexible such that they will reversibly bend or twist to a point where their differing flexibilities lead to loss of alignments of the cutting cannula and stylet at the distal end of the flexible biopsy needle. A biopsy needle fabricated with more flexibility and length, results in lower pushability and torquability.

Thus, there is a need for a transvenous biopsy device that can be introduced into the peripheral venous system of the arm into the patient's body via the basilica/cephalic veins, for example, and flexibly navigated through the venous system for performing a soft tissue biopsy on a target organ, such as the liver, so as to reduce the risk of major complications associated with PLB and TJLB procedures.
In patent application no. US 2015/141836 an intravascular ultrasound "(IVUS") device having an intrinsic or attachable needle guide is disclosed. This document describes a sheath having a needle guide used in connection with the IVUS device, to perform minimally invasive image-guided surgical procedures. The devices may be configured to maintain a needle placed through guide in the plane of the IVUS-array to improve visualization of the needle. However, the needle of this device cannot be pushed into liver parenchyma. At certain angles, the liver parenchyma at biopsy site cannot be entered without flopping.

### SUMMARY OF THE INVENTION

There is thus provided, in accordance with the present invention, a balloon-anchored, biopsy device for acquiring a biopsy sample of a target organ in a subject comprising the features defined in independent claim 1.

Furthermore, in accordance with some embodiments of the present invention, the flexible biopsy needle may include a Tru-cut biopsy needle.

Furthermore, in accordance with some embodiments of the present invention, the first elongated tube and the second elongated tube respectively may include a balloon catheter and a guide catheter.

Furthermore, in accordance with some embodiments of the present invention, the target organ may include a liver.

Furthermore, in accordance with some embodiments of the present invention, the blood vessel may include a hepatic vein of the liver.

Furthermore, in accordance with some embodiments of the present invention, the biopsy device may include a locking mechanism coupled to the second proximal end for fixing the position of the flexible biopsy needle at the distal end of the wire in the second lumen.

Furthermore, in accordance with some embodiments of the present invention, components of the locking mechanism may be selected from the group consisting of a Tuohy Borst adapter, a luer lock, and a compressible clamp.

Furthermore, in accordance with some embodiments of the present invention, the flexible biopsy needle may include a cutting cannula and a stylet.

Furthermore, in accordance with some embodiments of the present invention, the flexible biopsy needle may include a flattened band and an alignment notch for maintaining an alignment of the cutting cannula and the stylet.

Furthermore, in accordance with some embodiments of the present invention, the biopsy device may include an outer tube with the predefined length into which the first elongated tube and the second elongated tube are inserted so as to longitudinally attach the first elongated tube and the second elongated tube to one another.

Furthermore, in accordance with some embodiments of the present invention, the diameter of the inflated balloon is larger than the diameter of the blood vessel.

Furthermore, in accordance with some embodiments of the present invention, the diameter of the inflated balloon is no larger than 20% of the diameter of the blood vessel.

Furthermore, in accordance with some embodiments of the present invention, the predefined angle is in the range of 15-45 degrees.

Furthermore, in accordance with some embodiments of the present invention, the biopsy device may include a connecting tube for insertion into the second lumen for guiding the flexible biopsy needle at the distal end of the wire to the biopsy site.

Furthermore, in accordance with some embodiments of the present invention, the flexible biopsy needle may include a stylet joined to a stylet wire in an end-to-end joint.

Furthermore, in accordance with some embodiments of the present invention, the flexible biopsy needle may include a hollow stylet and a stylet wire inserted into an overlapping joint.

Furthermore, in accordance with some embodiments of the present invention, the flexible biopsy needle may include an inner stylet and outer stylet with a cutting edge arranged in a concentric configuration.

Furthermore, in accordance with some embodiments of the present invention, the flexible biopsy needle is configured to acquire the biopsy sample by rotating the outer stylet with the cutting edge relative to the inner stylet when the flexible biopsy needle is within the tissue of the target organ.

Furthermore, in accordance with some embodiments of the present invention, the flexible biopsy needle is configured to encapsulate the acquired biopsy sample in a specimen notch when the outer stylet remains in a rotated position substantially opposite to the inner stylet.

Furthermore, in accordance with some embodiments of the present invention, the biopsy device may include a rigid contoured section coupled to the distal end of the second elongated tube for increasing the predefined angle when the balloon is inflated.

Furthermore, in accordance with some embodiments of the present invention, the balloon may include a distal balloon and a proximal balloon, which are inflatable separately or together in the blood vessel.

Furthermore, in accordance with some embodiments of the present invention, the second elongated tube may be coupled to a pressure transducer for measuring a hepatic venous pressure gradient (HVPG) by processing a signal from the pressure transducer in a signal processing unit.

There is further provided, in accordance with some non-claimed examples, a method for acquiring a biopsy sample of a target organ of a subject using a balloon-anchored biopsy device may include percutaneously inserting a biopsy device into a vein of a limb of a subject, the biopsy device including a first elongated tube, a second elongated tube, and a flexible biopsy needle. The first elongated tube may enclose a first lumen with a first proximal end and a distal tip, wherein a section of the first elongated tube near the distal tip may include a balloon that when inserted into a blood vessel of a target organ of a subject and inflated, anchors the section in the blood vessel near a biopsy site in the target organ. The second elongated tube may enclose a second lumen with a second proximal end and a second distal end may include a beveled distal exit of the second lumen, which is positioned at the biopsy site of the target organ when the first elongated tube is anchored in the blood vessel by the inflated balloon, wherein a predefined length of the first and the second elongated tubes are longitudinally attached to one another such that the beveled distal exit is positioned at a proximal end of the section of the first elongated tube. The flexible biopsy needle may be attached to a distal end of a wire for insertion into the second lumen of the second elongated tube for navigation to the biopsy site, wherein the flexible biopsy needle is configured to exit the beveled distal exit of the second lumen for penetration into tissue of the target organ at the biopsy site at a predefined angle between a longitudinal axis of the section of the first elongated tube and a longitudinal axis of the flexible biopsy needle. The distal tip may be navigated from the vein through a vascular system of the subject and into the blood vessel of the target organ near the biopsy site. The balloon may be inflated in the blood vessel. The flexible biopsy needle may be pushed into the tissue of the target organ at the biopsy site at the predefined angle. A biopsy sample of the target organ at the biopsy site may be acquired using the flexible biopsy needle. The wire may be withdrawn from the second lumen so as to retrieve the acquired biopsy sample.

Furthermore, in accordance with some non-claimed examples, the limb may include an arm of the subject and the vein may include a cephalic vein of the arm.

Furthermore, in accordance with some non-claimed examples, the limb may include a leg of the subject and the vein may include a femoral vein of the leg.

Furthermore, in accordance with some non-claimed examples, percutaneously inserting the biopsy device into the vein of the limb of the subject may include inserting the biopsy device through a lumen of a sheath in the vein.

Furthermore, in accordance with some non-claimed examples, the balloon may include a distal balloon and a proximal balloon, and the method may include inflating the balloon comprises inflating the distal balloon and the proximal balloon separately or together in the blood vessel.

Furthermore, in accordance with some non-claimed examples, the second elongated tube may be coupled to a pressure transducer, and the method may include measuring a hepatic venous pressure gradient (HVPG) by processing a signal from the pressure transducer.

### BRIEF DESCRIPTION OF THE DRAWINGS

In order for the present invention, to be better understood and for its practical applications to be appreciated, the following Figures are provided and referenced hereafter. It should be noted that the Figures are given as examples only and in no way limit the scope of the invention. Like components are denoted by like reference numerals.
Fig. 1 schematically illustrates a long, flexible Tru-cut biopsy needle;
Fig. 2A schematically illustrates optimal alignments of a cutting cannula with a stylet of a non-flexible Tru-cut biopsy needle in a closed configuration;
Fig. 2B schematically illustrates optimal alignments of a cutting cannula with a stylet of a non-flexible Tru-cut biopsy needle in an open configuration;
Fig. 3 schematically illustrates a liver biopsy procedure performed on a subject with a balloon-stabilized biopsy device, in accordance with some embodiments of the present invention;
Fig. 4A schematically illustrates a balloon-stabilized catheter body, in accordance with some embodiments of the present invention;
Fig. 4B schematically illustrates a second embodiment of balloon-stabilized catheter body, in accordance with some embodiments of the present invention;
Fig. 4C schematically illustrates a two-sectioned inflatable balloon, in accordance with some embodiments of the present invention;
Fig. 5 schematically illustrates a longitudinal cross-sectional view of a long, flexible Tru-cut needle with distal self-alignment, in accordance with some embodiments of the present invention;
Fig. 6 schematically illustrates a first embodiment of a handle for use with flexible Tru-cut needle, in accordance with some embodiments of the present invention;
Fig. 7A schematically illustrates a balloon-stabilized biopsy device with a retracted flexible Tru-cut needle for navigation, in accordance with some embodiments of the present invention;
Fig. 7B schematically illustrates a balloon-stabilized biopsy device with an extended flexible Tru-cut needle for soft tissue acquisition, in accordance with some embodiments of the present invention;
Fig. 7C schematically illustrates a second embodiment of a balloon-stabilized biopsy device with a retracted flexible Tru-cut needle for navigation, in accordance with some embodiments of the present invention;
Fig. 7D schematically illustrates a second embodiment of a balloon-stabilized biopsy device with an extended flexible Tru-cut needle for soft tissue acquisition, in accordance with some embodiments of the present invention;
Fig. 8A schematically illustrates a distally-aligned flexible Tru-cut needle in a closed configuration, in accordance with some embodiments of the present invention;
Fig. 8B schematically illustrates a distally-aligned flexible Tru-cut needle in an open configuration, in accordance with some embodiments of the present invention;
Fig. 9 schematically illustrates a handle adapter with a middle shaft, in accordance with some embodiments of the present invention;
Fig. 10 schematically illustrates a second embodiment of a distal section of a balloon catheter, in accordance with some embodiment of the present invention;
Fig. 11A schematically illustrates a solid stylet joined end-to-end with a stylet wire, on accordance with some embodiments of the present invention;
Fig. 11B schematically illustrates a hollow stylet overlappingly joined with stylet wire, on accordance with some embodiments of the present invention;
Fig. 12A schematically illustrates a torque-based cut biopsy needle with an outer stylet and an inner stylet, in accordance with some embodiments of the present invention;
Fig. 12B schematically illustrates a top view of torque-based cut biopsy needle 500 in an open configuration and an encapsulation configuration, in accordance with some embodiments of the present invention;
Fig. 12C schematically illustrates rotating an outer stylet grip for performing a torque-based cut needle biopsy, in accordance with some embodiments of the present invention;
Fig. 13A schematically illustrates an exploded view of a second embodiment of a handle, in accordance with some embodiments of the present invention;
Fig. 13B schematically illustrates handle before firing, in accordance with some embodiments of the present invention;
Fig. 13C schematically illustrates handle after firing, in accordance with some embodiments of the present invention;
Fig. 14A schematically illustrates an adapter for coupling a balloon-stabilized catheter body to a hepatic venous pressure gradient (HVPG) measurement system, in accordance with some embodiments of the present invention; and
Fig. 14B is a graph of a hepatic venous pressure gradient (HVPG) measurement, in accordance with some embodiments of the present invention.

### DETAILED DESCRIPTION OF THE INVENTION

In the following detailed description, numerous specific details are set forth in order to provide a thorough understanding of the invention. However, it will be understood by those of ordinary skill in the art that the invention may be practiced without these specific details. In other instances, well-known methods, procedures, components, modules, units and/or circuits have not been described in detail so as not to obscure the invention.

Although embodiments of the invention are not limited in this regard, discussions utilizing terms such as, for example, "processing," "computing," "calculating," "determining," "establishing", "analyzing", "checking", or the like, may refer to operation(s) and/or process(es) of a computer, a computing platform, a computing system, or other electronic computing device, that manipulates and/or transforms data represented as physical (e.g., electronic) quantities within the computer's registers and/or memories into other data similarly represented as physical quantities within the computer's registers and/or memories or other information non-transitory storage medium (e.g., a memory) that may store instructions to perform operations and/or processes. Although embodiments of the invention are not limited in this regard, the terms "plurality" and "a plurality" as used herein may include, for example, "multiple" or "two or more". The terms "plurality" or "a plurality" may be used throughout the specification to describe two or more components, devices, elements, units, parameters, or the like. Unless explicitly stated, the method embodiments described herein are not constrained to a particular order or sequence. Additionally, some of the described method embodiments or elements thereof can occur or be performed simultaneously, at the same point in time, or concurrently. Unless otherwise indicated, use of the conjunction "or" as used herein is to be understood as inclusive (any or all of the stated options).

Embodiments of the present invention herein describe a balloon-stabilized core needle biopsy device for peripheral access (e.g., transcephalic access) that overcomes problems of insufficient force transfer from the handle to the cutting cannula and stylet at the biopsy site as well as the loss of alignment between the cutting cannula and the stylet for long, flexible Tru-cut needles. The balloon-stabilized core needle biopsy device includes an endovascular in vivo stabilizing system (e.g., a dual catheter body with an inflatable balloon anchor) and a distally self-aligned flexible Tru-cut needle.

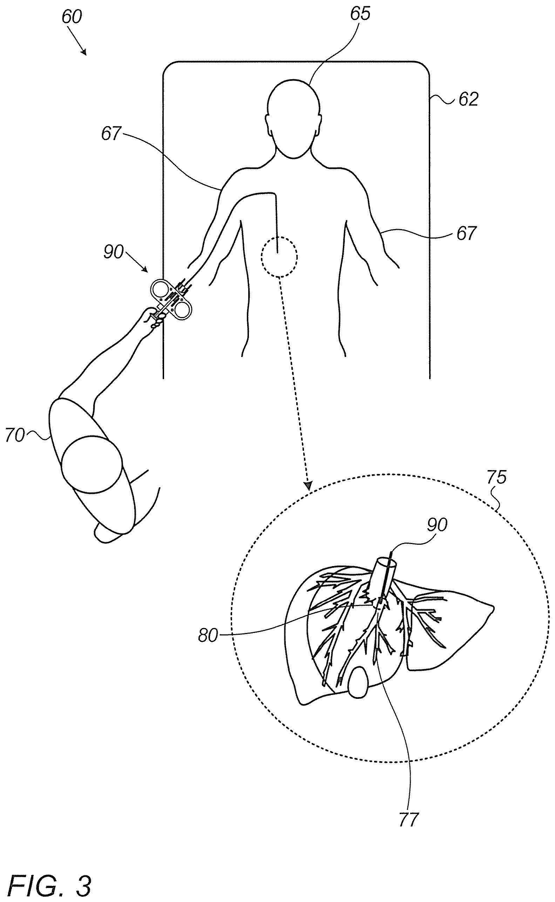

Fig. 3 schematically illustrates a liver biopsy procedure 60 performed on a subject 65 with a balloon-stabilized biopsy device 90, in accordance with some embodiments of the present invention. A doctor 70 may percutaneously insert a distal end of balloon-stabilized biopsy device 90 into an arm 67 of subject 65 lying on a gurney 62. Doctor 70 may navigate balloon-stabilized biopsy device 90 through the venous system of subject 65 into a hepatic vein 77, such as a middle hepatic vein, for example, to a biopsy target site 80 of a liver 75.

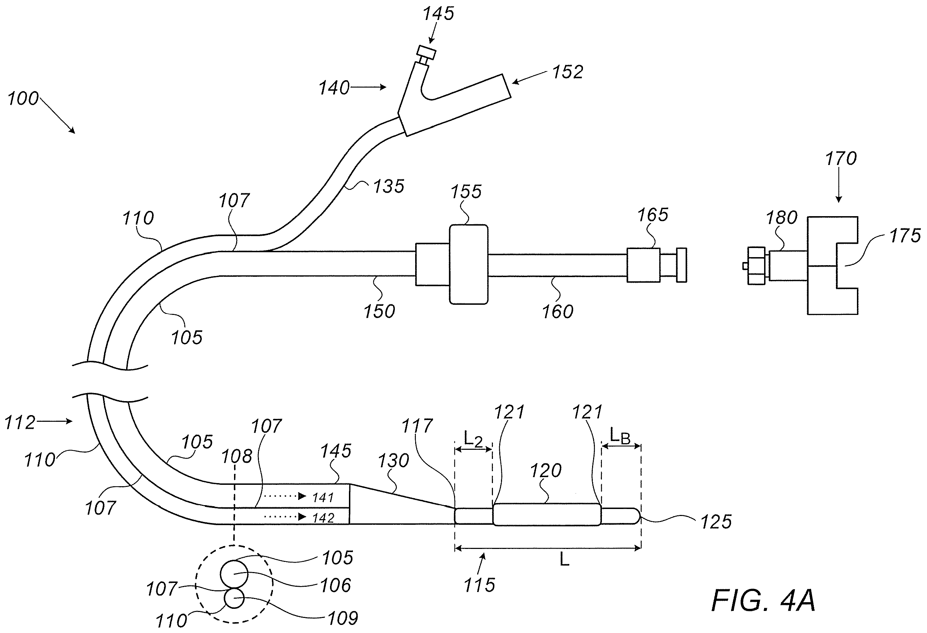

Fig. 4A schematically illustrates balloon-stabilized catheter body 100, in accordance with some embodiments of the present invention. Balloon-stabilized catheter body 100 includes a guide catheter 105, a balloon catheter 110, a beveled distal exit 130, and an inflatable (semi-compliant) balloon 120 with angled edges 121. Inflatable balloon 120 may be placed at a section 115 of balloon catheter 110 of length L and positioned a predefined distance from a distal tip 125 (e.g. denoted L_{B}). If the peripheral route for inserting balloon-stabilized catheter body 100 into the body of subject 65 is transcephalic (e.g., in Fig. 3), or transfemoral, the overall profile of the balloon-stabilized catheter body should be kept smaller than 5mm.

In some embodiments of the present invention, the outer walls of guide catheter 105 and balloon catheter 110 may be longitudinally attached 107 to one another over any suitable predefined length of balloon-stabilized catheter body 100. Stated differently, attachment curve 107 between guide catheter 105 and balloon catheter 110 may be substantially parallel to the longitudinal axes (e.g., 141 and 142) of those catheters through the respective catheter lumens when attached.

In some embodiments this may be enabled by fabricating a dual lumen catheter body. In other embodiments, an outer tube may be used to hold guide catheter 105 and balloon catheter 110 longitudinally attached 107 to one another over any suitable portion of the length of balloon-stabilized catheter body 100. Any suitable means may be used to hold guide catheter 105 and balloon catheter 110 longitudinally attached 107 to one another, such that guide catheter 105 and balloon catheter 110 move together with one another when balloon-stabilized catheter body 100 may be navigated through the venous system. A cross-sectional cut 108 of schematically illustrates a lumen 106 of guide catheter 105 and a lumen 109 of balloon catheter 110.

In some embodiments, balloon-stabilized catheter body 100 may include a handle adapter 170, which is configured to enable the handle of any Tru-cut needle (e.g. handle 15, for example) to be coupled securely to the stabilizing system.

Balloon-stabilized catheter body 100 may be also referred to herein as a "catheter body", a "balloon-anchored catheter body", or a "stabilizing system". Similarly, balloon-stabilized biopsy device 90 may be referred to herein as a "balloon-stabilized biopsy device", or a "balloon-anchored biopsy device" which may include balloon-stabilized catheter body 100 and a biopsy needle including the associated biopsy needle/stylet wire inserted through balloon-stabilized catheter body 100 to the target organ, such as liver 75, and used to acquire the soft tissue biopsy sample from the target biopsy site.

In some embodiments of the present invention, balloon 120 may be fabricated with a semi-compliant construction with a length no longer than 20mm. A proximal end 135 of balloon catheter 110 may include a hub 140, common to over-the-wire balloon catheters. Hub 140 may include an inflation port 145 and a guidewire inlet/exit port 152. Balloon 120 may be inflated by air coupled into inflation port 145. Any suitable air valve or stopper mechanism, for example, may be used to hold the air within balloon 120 to keep it inflated or opened to deflate balloon 120.

Section 115 of balloon catheter 110 of a length L, typically 20 mm, may be configured to be inserted into a blood vessel of the target organ, such as the hepatic vein of the liver, for example. Section 115 of balloon catheter 110 terminates in distal tip 125. Once inserted, balloon 120 may be inflated so as to anchor section 115 of balloon catheter 110 within the blood vessel. The maximum balloon diameter may be sized to be no larger than the target vessel diameter by a factor of 20%. (See, for example, Cook Medical G26902 - Advance ATB Percutaneous Transluminal Angioplasty (PTA) Dilation Catheter, whose specification is disclosed herein by reference. The length of the Cook Medical G26902 catheter is 80 cm with an inflated balloon diameter of 8 mm.)

The shaft of guide catheter 150 may be formed from a braid-reinforced polymer tube, typically at least 700mm in length, with a maximum distal curvature 112 of about 60° so as permit the Tru-cut needle to pass through guide catheter 105 in the tortuous regions of the venous system, such as through brachiocephalic/superior vena cava junction, for example. A distal end 145 of the guide catheter 105 may be terminated with a beveled distal exit 130, where a longer edge of beveled distal exit 130 may terminate at a proximal end 117 of the balloon catheter shaft (e.g., section 115) at 5 - 10mm proximal (e.g. L₂) of inflatable balloon 120. The bevel angle may be between 15 - 45°. Beveled distal exit 130 may be formed by melting a polymer tube, whose inner diameter may be slightly larger than the combined diameters of balloon catheter 110 and guide catheter 105, over distal end 145 of guide catheter 105 and balloon catheter 110. The melted polymer tube may then be skived to shape to form beveled distal exit 130.

A proximal end 150 of guide catheter 105 may be joined to a Tuohy Borst adapter 155, which when fully loosened, allows free passage of a braid-reinforced polymer connecting tube 160 through Tuohy Borst adapter 155. Connecting tube 160 may have outer diameter sized to fit smoothly into guide catheter 105. Once Tuohy Borst adapter 155 may be sufficiently tightened, it locks connecting tube 160 firmly in place, thus preventing any further axial movement. The proximal end of connecting tube 160 is joined to a luer lock 165. The inner diameters of luer lock 165 and connecting tube 160 may be sized to allow free movement of the desired long, flexible cut biopsy needle within them.

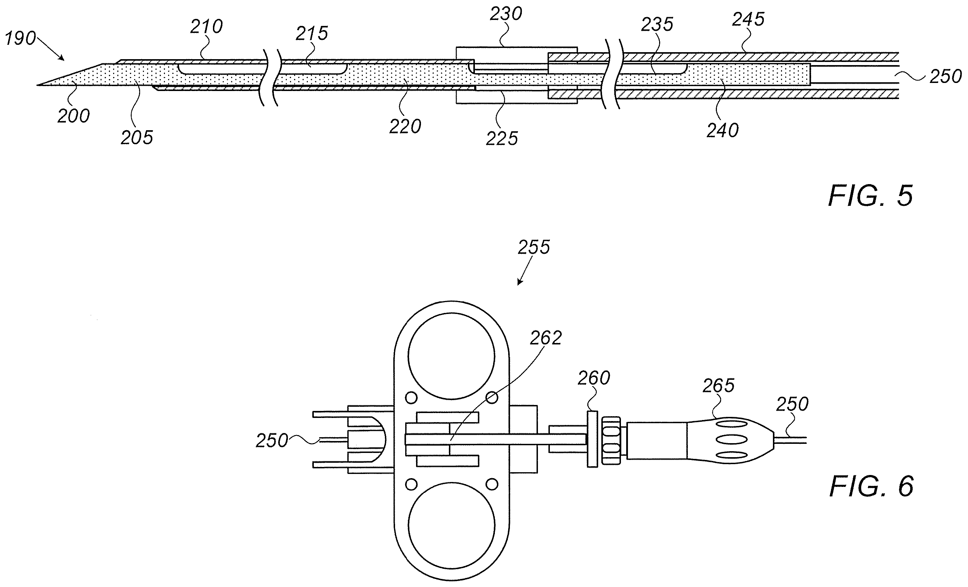

A second luer lock 180 with a spin lock connector, complementary to luer lock 165 of connecting tube 160, may be present on the distal end of Tru-cut needle handle 15 for example, (or additional embodiments of handles shown herein) such that when the luer locks are connected, Tru-cut needles may be securely fastened to balloon-stabilized catheter body 100. Typically, Tru-cut needle handles 15 are not be fabricated with such a second luer lock 180 with spin lock connector at its distal end, thus a handle adapter 170 may be used. Handle adapter 170 may include second luer lock 180 with spin lock connector for coupling to luer lock 165 of connecting tube 160. Handle adapter 170 may include on its proximal end a clamp 175 customized to fit with the desired Tru- cut needle handle 15. The inner diameter of second luer lock 180 may be sized to allow free passage of a cutting cannula and cutting cannula tube as in Fig. 5 below.

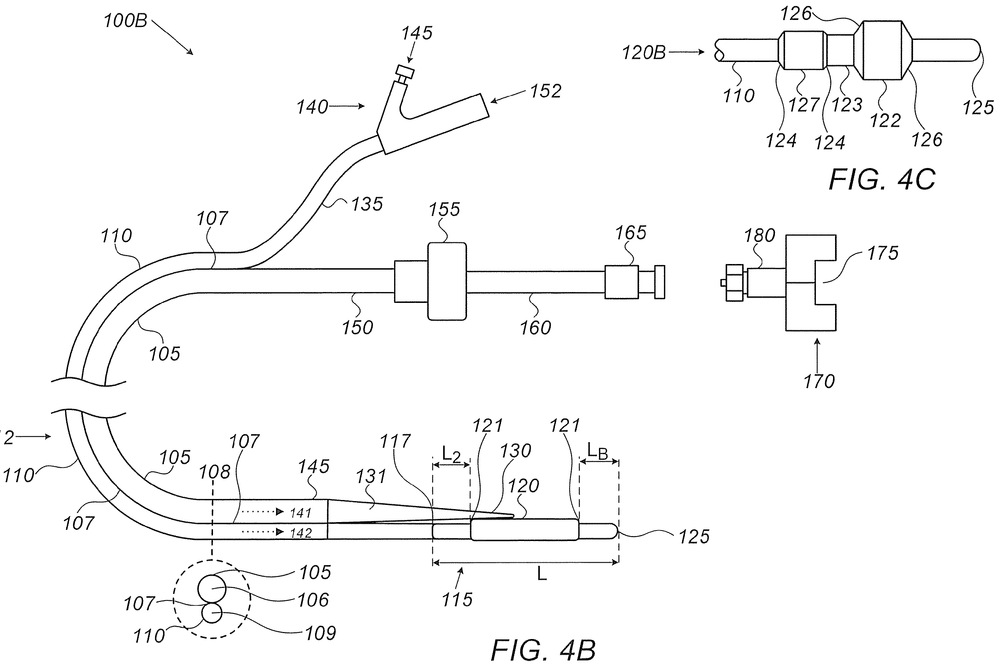

Fig. 4B schematically illustrates a second embodiment of a balloon-stabilized catheter body 100B, in accordance with some embodiments of the present invention. The region near distal tip 125 of balloon-stabilized catheter body 100B may include a rigid contoured section 131 of guide catheter 105 such that beveled distal exit 130 may be positioned over inflatable balloon 120 and not in front of inflatable balloon 120 as shown in Fig. 4A.

Fig. 4C schematically illustrates a two-sectioned inflatable balloon 120B, in accordance with some embodiments of the present invention. Two-sectioned inflatable balloon 120B positioned near distal tip 125 of balloon catheter 110 may include a proximal balloon 127 with angled edges 123 and a distal balloon 122 with angled edges 126 with an intermediate section 123 between proximal balloon 125 and distal balloon 122. In some embodiments, proximal balloon 125 and distal balloon 122 may be inflated separately or inflated together. In other embodiments, two-sectioned inflatable balloon 120B may be used in Figs. 4A and 4B in place of inflatable balloon 120. Two-sectioned inflatable balloon 120B may have a dog-bone shape, which provides better anchoring in the hepatic vein. Distal balloon 122 may be positioned at the beginning of the hepatic vein and may be used to control how far into the liver the biopsy device may go. Over insertion of the biopsy device into the liver may pose a risk that stylet 200 may pierce through the other side of the liver.

Fig. 5 schematically illustrates a longitudinal cross-sectional view of a long, flexible Tru-cut needle 190 with distal self-alignment, in accordance with some embodiments of the present invention. Flexible Tru-cut needle 190 with distal self-alignment may include a cutting cannula 210 and a stylet 200. Stylet 200 may have a tubular form with notches in two separate sections: a specimen notch 215 and an alignment notch 235. Specimen notch 215 in a stylet distal section 205 of flexible Tru-cut needle 190 may be used to hold the soft tissue biopsy specimen after shearing the soft tissue of the target organ with cutting cannula 210. Alignment notch 235 may be located in a stylet proximal section 240 of flexible Tru-cut needle 190.

In some embodiments of the present invention, the distal self-alignment of flexible Tru-cut needle 190 may be enabled as follows. Stylet 200 may be threaded through a flattened band 225, where flattened band 225 may begin as a circular band of the same outer and inner diameters as cutting cannula 210, threaded over alignment notch 235, then progressively flattened until stylet 200 is unable to rotate about alignment notch 235. However, stylet 200 may be free to move longitudinally along alignment notch 235, but bound by a stylet middle section 220 and stylet proximal section 240.

Flattened band 225 may be bonded at its distal end to the proximal end of cutting cannula 210, and at its proximal end to the distal end of cutting cannula tube 245. Such a bonding 230 may be enabled using conventional fabrication techniques, such as melting an overtube or using heat shrink tubing reinforced with medical grade adhesives. Bonding 230 may be strengthened by using overtube or heat shrink tubing of sufficiently high hardness (>70D), increasing the roughness of the proximal end of cutting cannula 210 (e.g., by sandblasting), and using cutting cannula tube 245 with a coiled wire construction where the overtube can melt into the grooves of the coil, for example.

Fig. 6 schematically illustrates a first embodiment of a handle 255 for use with flexible Tru-cut needle 190, in accordance with some embodiments of the present invention. The handle 255 is similar to handle 15 of Fig. 1 with the exception of changes in an actuator 260 and the addition of a pin vise 265 at the proximal end of actuator 260. A stylet wire 250 may be threaded through to extend out of pin vise 265. When pin vise 265 is loosened, stylet wire 250 may be free to slide longitudinally as well as rotate. However, once pin vise 265 is tightened, stylet wire 250 may be firmly held in position by pin vise 265. Actuator 260 may not be bonded to stylet wire 250, as in Fig. 1, for example, but instead includes a hole that allows stylet wire 250 to pass through unrestricted. The rest of the structure and functionality of handle 255 may be similar to handle 15 as shown in Fig. 1. Cocking the spring-loaded firing mechanism in handle 255 may be enabled by operator 70 pulling back actuator 260 until carriage may be caught in a catch 262.

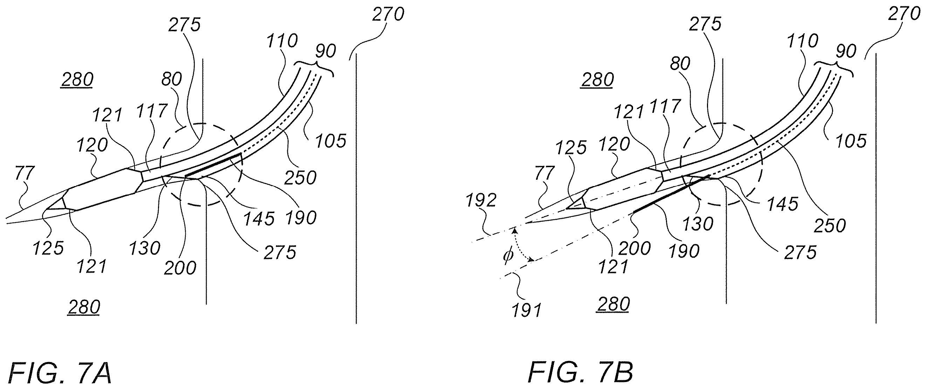

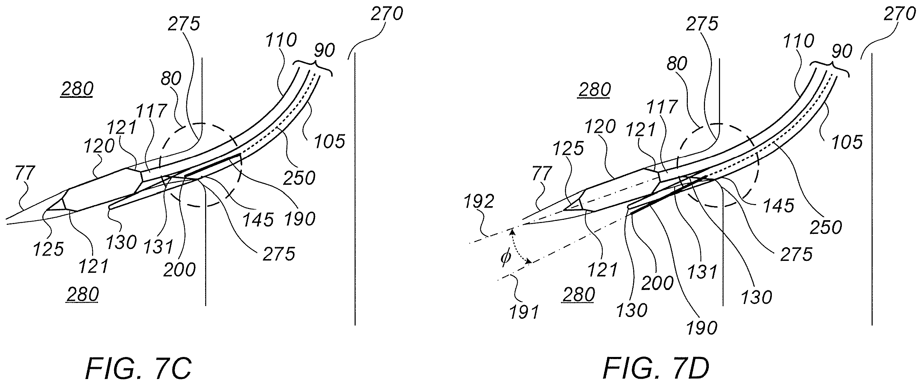

In the context of this disclosure, the term flexible Tru-cut needle 190 as used herein may not only refer to the stylet apparatus shown in Fig. 7, but may also include any suitable wire such as stylet wire 250, for example, connected at the wire's proximal end to handle 250 and to stylet 200 at the wire's distal end wherein the stylet end of flexible Tru-cut needle 190 may be fed through proximal end 150 of catheter 105, for example.

Methods for performing a liver biopsy using balloon-stabilized biopsy device 90 introduced into arm 67 of subject 65 and navigated to a target organ, such as liver 75, via a transcephalic route are described herein below in accordance with some embodiments of the present invention. An operator, such as doctor 70, may connect preloaded flexible Tru-cut needle 190 to handle adapter 130 via customized clamp 175. Operator 70 may then insert flexible Tru-cut needle 190 into connecting tube 160, and may use luer lock 165 of connecting tube 160 and second luer lock 180 of handle adapter 130 to lock the position of flexible Tru-cut needle 190. Tuohy borst adapter 155 may be loosened, and connecting tube 160 with locked Tru-cut needle 190 may be inserted into the shaft of guide catheter 105.

Connecting tube 160 may be slid through guide catheter 105 until stylet 200 may be positioned, but safely held within guide catheter distal end 145. The tip of stylet 200 may be held in guide catheter distal end 145 at a distance of 5 mm, for example, from beveled distal exit 130. Touhy borst adapter 155 may then be locked to keep the connecting tube with flexible Tru-cut needle 190 in place with respect to the shaft of guide catheter 105.

In some embodiments of the present invention, operator 70 may gain access to the venous system by placing an introducer sheath in arm 67 of subject 65. The introducer sheath may then be exchanged for balloon-stabilized biopsy device 90. In this step, venous access of balloon-stabilized biopsy device 90 may be aided by its streamlined profile provided by deflated balloon 120 and beveled distal exit 130. Balloon-stabilized biopsy device 90 may be introduced into a lumen of the introducer sheath and into the venous system.

In some embodiments of the present invention, the introducer sheath may be elongated so that it reaches beyond the first bend where the subclavian vein joins with the superior vena cava above the heart. The introducer sheath may be armored, such as by using a coil-wire design. The introducer sheath may not be removed, but left in place and connected by an interference fit for providing additional stability on the other end. The introducer sheath may extend protection and improved trackability around the first and most acute bend (e.g., subclavian to superior vena cava) from the biopsy needle.

Fig. 7A schematically illustrates balloon-stabilized biopsy device 90 with a retracted flexible Tru-cut needle 190 for navigation, in accordance with some embodiments of the present invention.

Fig. 7B schematically illustrates balloon-stabilized biopsy device 90 with an extended flexible Tru-cut needle 190 for soft tissue acquisition, in accordance with some embodiments of the present invention.

Balloon-stabilized biopsy device 90 may be navigated through the venous system to liver 75, For example, operator may introduce balloon-stabilized biopsy device 90 into the basilic/cephalic vein in arm 67. Balloon-stabilized biopsy device 90 may then be navigated through the brachiocephalic/superior vena cava junction into the superior vena cava, through the heart into inferior vena cava 270 and finally into hepatic vein 77, such as the middle hepatic vein, for example. The navigation of distal tip 125 of balloon-stabilized biopsy device 90 may be further enabled using guidewire support in balloon catheter 110 until beveled distal exit 130 may no more than 15mm distal of a hepatic vein ostium 275. Throughout the navigation and tracking step, sharp stylet 200 may not injure the veins during tracking because stylet 200 may be kept safety within the shaft of guide catheter 105 near guide catheter distal end 145 as shown in Fig. 7A.

Once beveled distal exit 130 is positioned at biopsy target site 80 with section 115 of balloon catheter 110 in hepatic vein 77, balloon 120 may inflated to a diameter (e.g., up to 20% larger than the vein diameter) so as to anchor section 115 of balloon catheter 110 in hepatic vein 77. At this point, the guidewire in balloon catheter 110 may be left in position, so as to provide additional stability to the system. Since beveled distal exit 130, from where cutting cannula 210 and stylet 200 exit is proximal to inflated balloon 120, cutting cannula 210 and stylet 200 should be directed obliquely towards liver parenchyma 280, instead of co-axially with hepatic vein 77 towards balloon 120.

To achieve this, balloon catheter 110 is designed to be more flexible than guide catheter 105, which enables guide catheter distal end 145 to tilt relative to a longitudinal axis 192 of section 115 (see Fig. 4A) of balloon catheter 110 with inflated balloon 120 and to remain fixed, and not co-axial or parallel with hepatic vein 77, as shown in Figs. 7A and 7B. As a result, stylet 200 of flexible Tru-cut needle 190 may be pushed into liver parenchyma 280 at an angle ϕ between a longitudinal axis 191 of flexible Tru-cut needle 190 and longitudinal axis 192 of section 115 (see Fig. 4A) with balloon 120 as shown in Fig. 7B. ϕ may be in the range of 15-45 degrees. In this configuration, stylet 200 may cleanly enter liver parenchyma 280 at biopsy site 80 without flopping around as described previously for the non-anchored or stabilized case. Moreover, this oblique configuration may prevent stylet 200 from popping inflated balloon 120. In some embodiments, more flexibility of balloon catheter 110 may be achieved by reducing the diameter of balloon catheter 110 from 5.0 Fr to 4.0 Fr on the French scale.

In some embodiments, the outer walls of guide catheter 105 and balloon catheter 110 may no longer be longitudinally attached 107 in the region of balloon-stabilized catheter body 100 near to guide catheter distal end 145 so as to facilitate achieving the oblique configuration with predefined angle ϕ as shown in Fig. 7B.

In some embodiments of the present invention, once balloon-stabilized biopsy device 90 and retracted flexible Tru-cut needle 190 are positioned at biopsy target site 80, operator 70 may then loosen Tuohy borst adapter 155. Connecting tube 160 and Tru-cut needle 190 may be pushed forward out of the shaft of guide catheter 105 until extended stylet 200 and cutting cannula 210 fully enters liver 75. Once cutting cannula 210 and stylet 200 have entered the tissue of liver 75, operator 70 may then tighten Tuohy borst adapter 155 to hold connecting tube 155 with handle 255 in place.

From this point onward, in some embodiments, operator 70 may perform the biopsy by firing the spring-loaded mechanism to drive cutting cannula 210 in liver parenchyma 280 toward stylet 200 (e.g., as any Tru-cut needle is operated). If the liver is not anchored, the liver may move forward if the stylet is slowly advanced, reducing the chance of a successful biopsy. In other embodiments, both the stylet and the cutting cannula may be spring loaded. They may be separately fired or fired together. The stylet may be fired followed by the cutting cannula in quick succession, which increases the chance of a successful liver biopsy.

If flexible Tru-cut needle 190 is fired at this point on its own without the balloon-anchored stabilization system, cutting cannula tube 245 will whip around starting from the cutting cannula tube proximal end just bonded to the carriage, because of the cutting cannula tube's flexibility. As a result of this whipping, most of the energy provided by the spring loaded mechanism in handle 255 that is meant to be transferred to cutting cannula 210 via cutting cannula tube 245 will be dissipated away before reaching the cutting cannula 210, leading to biopsy failure.

The balloon-anchored stabilizing system may significantly reduce this whipping effect through several means. Firstly, guide catheter 105 may provide support to cutting cannula tube 245 by encasing it in-vivo in a non-compliant, close fit environment such that the flexibility of cutting cannula tube 245 may be inhibited in the transverse direction, thereby improving its pushability, so long as cutting cannula tube 245 has sufficient column strength to withstand the energy from the spring-loaded firing mechanism in handle 255. Secondly, the securing the shaft of guide catheter 105, connecting tube 160, and handle adapter 130 provides additional support of cutting cannula tube 245 ex vivo. Thirdly, any recoil experienced by flexible Tru-cut needle 190 during its operation will not dislodge the stabilizing system due to the anchorage provided by inflated balloon 120, thereby increasing the efficiency of the support provided by guide catheter 105. Once cutting cannula tube whipping is reduced significantly, cutting cannula tube 245 may perform its intended function of energy transfer from the spring-loaded firing mechanism in handle 255 to cutting cannula 210 so as to perform the biopsy.

Once the biopsy sample has been obtained, balloon 120 may be deflated to restore blood flow in hepatic vein 77. With balloon-stabilized biopsy device 90 left in place, luer lock 165 of connecting tube 160 and second luer lock 180 of handle adapter 130 are loosened, and flexible Tru-cut needle 190 may be retracted out of the body. Using methods normal to Tru-cut needles operation, operator 70 may expose stylet 200 to inspect the quality of the biopsy sample stored in specimen notch 215.

If subsequent biopsy passes are needed, balloon-stabilized catheter body 100 for each new pass may be withdrawn until beveled distal exit 130 may be located just outside biopsy site 80, leaving deflated balloon still inside hepatic 77. Flexible Tru-cut needle 190 may then be reinserted back into balloon-stabilized catheter body 100 and tracked back to biopsy site 80 to obtain subsequent biopsy samples by the methods previously described herein.

Fig. 7C schematically illustrates a second embodiment of a balloon-stabilized biopsy device with rigid contoured section 131 with a retracted flexible Tru-cut needle for navigation, in accordance with some embodiments of the present invention.

Fig. 7D schematically illustrates a second embodiment of a balloon-stabilized biopsy device with rigid contoured section 131 with an extended flexible Tru-cut needle for soft tissue acquisition, in accordance with some embodiments of the present invention.

In the second embodiment, rigid contoured section 131 of guide catheter 105 may include distal beveled 130 positioned over and resting on angled edge 121 over inflatable balloon 120. When the balloon is inflated, this configuration may be used to increase angle ϕ between longitudinal axis 191 of flexible Tru-cut needle 190 during biopsy acquisition and longitudinal axis 192 when flexible Tru-cut needle 190 is extended for soft tissue acquisition relative to the first embodiment shown in Figs 7A and 7B without rigid contoured section 131.

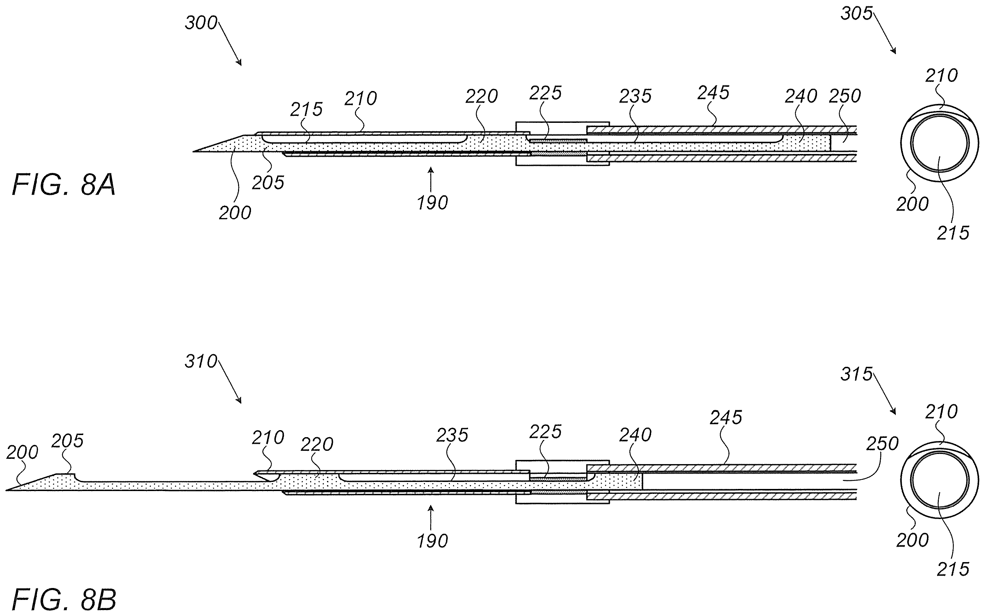

Fig. 8A schematically illustrates a distally-aligned flexible Tru-cut needle 190 in a closed configuration 300, in accordance with some embodiments of the present invention.

Fig. 8B schematically illustrates a distally-aligned flexible Tru-cut needle 190 in an open configuration 310, in accordance with some embodiments of the present invention.

The operation of long, flexible Tru-cut needle 190 is described hereinbelow in accordance with some embodiments of the present invention. Flexible Tru-cut needle 190 may be first laid out straight on sufficiently large surface, with pin vise 265 in handle 255 loosened. Cutting cannula 210 and stylet 200 may be aligned in the closed configuration as shown in Fig. 8A, with the corresponding position of flattened band 225 in alignment notch 235. Cocking the spring-loaded firing mechanism in handle 255 may be enabled by operator 70 pulling back actuator 260 until carriage may be caught in catch 262. As the carriage is being pulled back, the cutting cannula - flattened band - cutting cannula tube assembly may be pulled along in the same manner, whereby the existing friction between flattened band 225 and alignment notch 235 causes stylet 200 and cutting cannula 210 to retract as one unit, maintaining closed configuration 300. Once the spring is fully loaded, flexible Tru-cut needle 190 is ready for insertion into patient 65.

Operator 70 may manipulate the flexible Tru-cut needle 190 to desired biopsy site 80 through endovascular or endoscopic means in a tracking step. During this tracking step, stylet 200 and cutting cannula 210 should remain in the optimal closed configuration 300 for tracking. While tracking, both cutting cannula tube 245 and stylet wire 250 will be bending in accordance to how their differing flexibilities adapt to the tortuosity of the anatomy/working channel being navigated. As there is higher friction on Tru-cut needle distal section 205 between flattened band 225 and alignment notch 235 relative to the proximal end of balloon-stabilized biopsy device 90 where stylet wire 250 may freely move longitudinally about loosened pin vise 265, any bending differences between cutting cannula tube 245 and stylet wire 250 may be transferred to the proximal end of balloon-stabilized biopsy device 90 based on the path of least resistance. Therefore, this property of balloon-stabilized biopsy device 90 may ensure that cutting cannula 210 and stylet 200 may maintain their longitudinal alignment 305 of the closed configuration 300 throughout tracking.

In some embodiments to further ensure longitudinal alignment 305, the length of alignment notch 235 may be carefully controlled such that it corresponds closely to the expected movement distance of cutting cannula 210. For example, cutting cannula 210 with an allowed movement of 20 mm with flattened band 225 with a 3 mm length, the length of alignment notch 235 may be 25 mm. In this manner, even if stylet 200 may attempt to retract into cutting cannula 210 during tracking, the proximal end of stylet mid-section 220 may be blocked by flattened band 225 that is fixed by cutting cannula tube 245, thereby ensuring adequate longitudinal alignment 305.

Similarly, as flexible Tru-cut needle 190 is being manipulated to desired biopsy site 80 through endovascular or endoscopic means, both cutting cannula tube 245 and stylet wire 250 may be twisting in accordance with how their differing torquabilities react to the tortuosity of the anatomy/working channel being navigated. Since there is space at the distal end of flexible Tru-cut needle 190 for flattened band 225 to twist about alignment notch 235 as compared to the proximal end where stylet wire 250 may be free to rotate about loosened pin vise 265, any twisting differences between cutting cannula tube 245 and stylet wire 250 may be transferred to the proximal end based on the path of least resistance, therefore ensuring that cutting cannula 210 and stylet 200 maintain their rotational alignment 305 throughout tracking.

Once flexible Tru-cut needle 190 reaches desired biopsy site 80, operator 70 may advance Tru-cut needle 190 into the organ parenchyma, preferably until the tip of cutting cannula 210 may be safely inside the parenchyma. During this advancement step, the cutting cannula and stylet longitudinal alignment 305 should be maintained in the closed configuration 300 as shown in Figure 8A. This is achieved by the same alignment notch length control as previous described by way of example.

Once tip of cutting cannula 210 is inside the parenchyma, operator 70 may push out stylet 200 to open position 310 as shown in Fig. 8B, penetrating into the parenchyma of the target organ while maintaining the position of cutting cannula 210. During this stylet penetration step, the cutting cannula and stylet will reach a final longitudinal and rotational alignments 315 in open configuration 310 as shown in Fig. 8B.

Maintaining longitudinal alignment may also be enabled using alignment notch length control. Using the same lengths previously described of cutting cannula 210 that is allowed to move 20mm, a 3mm long flattened band and the corresponding alignment notch length of 25mm, the lengths may be designed such that when stylet 200 may be fully extended to final open configuration 310, the distal end of stylet proximal-section will be blocked by the proximal end of the flattened band, thereby preventing any over-extension away from the final longitudinal alignment.

The freedom of movement of the stylet wire proximal section by loosened pin vise 265 may also assist in achieving longitudinal alignment, as the pushability of stylet wire 250 may be overcome by allowing stylet wire 250 to be pushed as much as possible from outside patient 65 until the distal end of stylet proximal-section may be blocked by the proximal end of the flattened band, which may be tactilely sensed by operator 70 (e.g., tactile feedback) from stylet wire 250. With regards to rotational alignment, the close fit between flattened band 225 and alignment notch 235 throughout the stylet extension movement may ensure that both cutting cannula 210 and stylet 200 may be forced to turn and rotate as one single unit, thereby ensuring rotational alignment.

Once stylet 200 has been fully extended, operator 70 may tighten pin vise 265 to lock stylet wire 250 at its proximal end and fire the spring-loaded mechanism in handle 255 to drive cutting cannula 210 distally toward the tip of stylet 200 so as to cut and obtain the soft tissue specimen in the organ parenchyma. Note that locking stylet wire 250 via tightening pin vise 265 is crucial at this point as this ensures that the cutting cannula - flattened band - cutting cannula tube assembly may be propelled forward as one unit without stylet 200 being moved along with it. After the spring-loaded mechanism has been fired and the tissue specimen obtained in specimen notch 215, cutting cannula 210 and stylet 200 may be returned to the same longitudinal and rotational alignments in closed configuration 300, so that the tissue specimen within specimen notch 215 may be safely encapsulated between the cutting cannula 210 and stylet 200. This may be automatically ensured by careful control of the alignment notch length.

Furthermore using the same lengths described previously where cutting cannula 210 may move 20mm, 3mm long flattened band 225, and a length of alignment notch 235 of 25mm, these lengths may be designed such that once the stylet has been fully extended in the stylet penetration step, the cutting cannula distal end automatically ends up overlapping the stylet distal-section, achieving the encapsulation of the biopsy sample in specimen notch 215. The alignment notch length of 25mm, being longer than the combined cutting cannula and flattened band lengths of 23mm, also provides additional allowance for flattened band movement, such that the flattened band, when propelled by high speeds by the spring-loaded mechanism in handle 255, may not collide with the proximal end of stylet mid-section 220.

With the tissue specimen safely encapsulated, operator 70 may loosen pin vise 265 allowing free stylet wire movement, and may withdraw flexible Tru-cut needle 190 out of the patient's body to retrieve the sample. In this withdrawal step, the same design aspects of the flexible Tru-cut needle 190 as described previously in the tracking step may also be used to keep cutting cannula 210 and stylet 200 in the proper longitudinal and rotational alignments in closed configuration 300. Once flexible Tru-cut needle 190 may be withdrawn from the patient's body, operator 70 may extend the stylet to open configuration 310 for inspection and/or transfer of the tissue specimen out of specimen notch 215. If operator 70 deems that another biopsy pass must be performed, flexible Tru-cut needle 190 may be reset by pulling stylet 200 back to closed configuration 300, then pulling back actuator 260 to cock the spring-loaded firing mechanism.

In some embodiments of the present invention, a second method for using balloon-stabilized biopsy device 90 as a part of an endoscopic access to soft tissue/internal organs is described hereinbelow. Although ultrasound guided endoscopic transgastric access may be known, the use of Tru-cut biopsy needles within the endoscope has been documented to be technically challenging because of the stiffness of the biopsy needles. Thus, with the flexible, low profile and length of flexible Tru-cut needle 190 as described above, operator 70 may use it in conjunction with an endoscope, where the endoscope may be navigated through the gastro-intestinal system to a location closest to the target organ. Biopsy device 90 may be anchored by inflating the balloon, and firing the needle to pierce through the stomach/intestines directly into the target organ (such as the liver, kidney, etc.), so as to obtain the soft tissue biopsy sample.

In some embodiments of the present invention, during the venous access of balloon-stabilized biopsy device 90 in exchange with the introducer sheath, an additional long dilator may be sized to fit inside and throughout the shaft of guide catheter 105 so as to ease venous access. The dilator may be made in any suitable shape and construction.

In some embodiments of the present invention, although balloon-stabilized biopsy device 90 has been described herein for core needle biopsies, balloon-stabilized biopsy device 90 may be configured to work with fine needle aspiration biopsy systems, so long as the fine needle aspiration biopsy system is sufficiently long and flexible. This may be desirable for endovascular peripheral access for fine needle aspiration biopsy systems, as well as providing a stable platform for the fine needle aspiration biopsy systems.

In some embodiments of the present invention, although Tuohy borst adapter 155 may be used to control the mobility of the connecting tube as previously described, there are other methods to do so. For example, a compressible clip may be used to replace Tuohy borst adapter 155, where the compressible clip may be configured to clamp onto and to hold connecting tube 160 firmly in position. When operator 70 may compress the compressible clip, the clamping action may be removed so as to permit movement of connecting tube 160. In this manner, operator 70 may be free to adjust the longitudinal position of connecting tube 160. Once the desired positions of cutting cannula 210 and stylet 200 have been attained, operator 70 may release the compressible clip, so as to re-clamp and fix the position of connecting tube 160.

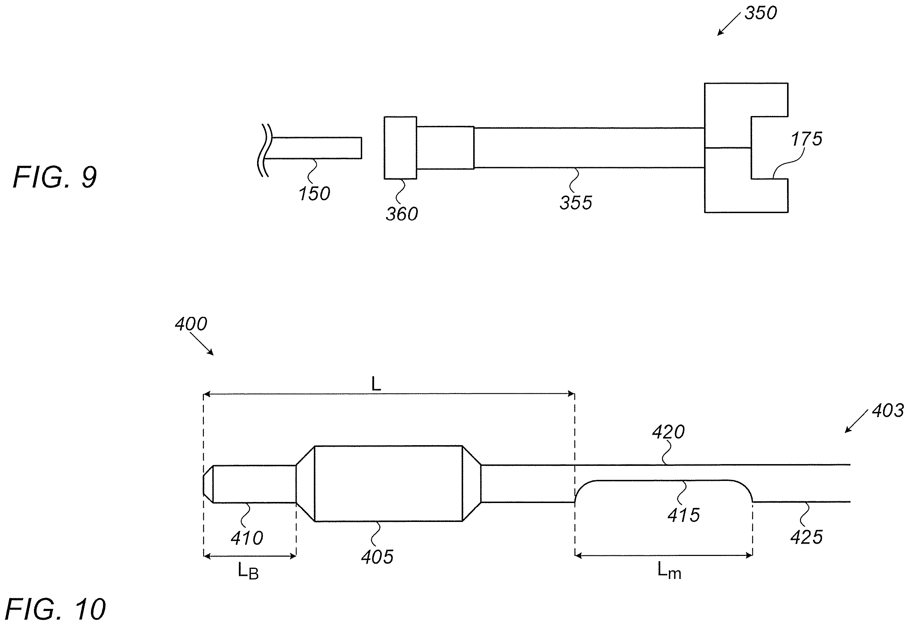

Fig. 9 schematically illustrates a handle adapter 350 with a middle shaft 355, in accordance with some embodiments of the present invention. In cases where no braid-reinforced connecting tube 160 may be used, a second embodiment of handle adapter 350 may be used, which is configured to directly receive guide catheter shaft proximal end 150. In this case, Tuohy borst adapter 155 of guide catheter shaft proximal end 150 may be first removed. Luer lock with spin connector of the handle adapter 180 may then be replaced by a second embodiment of a Tuohy borst adapter 360 that is large enough to accommodate easy passage of guide catheter shaft proximal end 150 when loosened. Next, handle adapter 350 may be lengthened by adding middle shaft 355 between Tuohy borst adapter 360 and customized clamp 175, where middle shaft 355 may include a sufficiently large inner diameter so as to allow free movement of guide catheter shaft proximal end 150 within it, so that the position of cutting cannula 210 and stylet 200 relative to guide catheter 105 may be adjusted. Tuohy borst adapter 360 may then be tightened so as to secure or fix guide catheter shaft proximal end 150 in the proper position.

In some embodiments of the present invention, braid-reinforced connecting tube 160 and handle adapter 130 may also be modified to become a retractable sheath for the Tru-cut needle when placed inside guide catheter 105. These modifications may include extending connecting tube 160 distally until it may sheath stylet 200, changing the construction of connecting tube 160 to a coil-reinforced polymer for added flexibility, and adding a retracting mechanism in handle adapter 130. In this manner, connecting tube 160 and flexible Tru-cut needle 190 may be inserted into balloon-stabilized catheter body 100 as a single unit. If additional biopsies are needed, only flexible Tru-cut needle 190 may be withdrawn without the need to withdraw any portions of balloon-stabilized catheter body 100. This method may prevent any potential damage to the shaft of guide catheter 105 by having to remove only connecting tube 160 from patient 65.

To perform the biopsy, connecting tube 160 may be retracted to unsheathe cutting cannula 210 and stylet 200 once the cutting cannula and stylet have reached desired biopsy target site 80. Tuohy borst adapter 155 joined to guide catheter shaft proximal end 150 may be tightened to lock the connecting tube in place, thereby firmly securing the connecting tube - handle adapter - Tru-cut needle to the stabilizing system of biopsy device 90.

Fig. 10 schematically illustrates a second embodiment of a section 400 of a balloon catheter 403, in accordance with some embodiment of the present invention. If a smaller profile (e.g., diameter of balloon-stabilized biopsy device 90) is desired, the guidewire lumen of balloon catheter 110 may be removed while keeping an inflation channel, with the trade-off that there will be no guidewire support for balloon-stabilized biopsy device 90. In this embodiment, an inflatable balloon 405 of section 400 of balloon catheter 403 of length L may be positioned at a predefined distance from a distal tip 410 (e.g. denoted L_{B}). At a proximal end of section 400, a mid-section 420 of length Lₘ may couple balloon catheter 403 to section 400 and include a distal exit 415.

The shaft section of balloon catheter 403 may include a braid-reinforced design so as to provide sufficient support for flexible Tru-cut needle 190. In the embodiment shown in Fig. 10, during tracking of flexible Tru-cut needle 190 to hepatic vein 77 of liver 75, for example, the guidewire may be threaded through section 400, mid-section 420, and shaft of catheter 403. After reaching hepatic vein 77 and inflating balloon 405 with mid-section 420 placed, for example, at hepatic vein ostium 275, the guidewire may be exchanged for flexible Tru-cut needle 190 with its own retractable sheath. Mid-section 420 may be configured to be more flexible than shaft section 425 of catheter 403 due to the cut-out section of a distal exit 415, being substantially more pliant than shaft 425 without the cut-out section. Mid-section 420 may then tilt relative to the longitudinal axis of section 400 anchored in hepatic vein 77. Flexible Tru-cut needle 190 (e.g., cutting cannula 210 and stylet 200) may exit from distal exit 415 at an angle ϕ as defined in Fig. 7B and may be directed into liver parenchyma 280 and away from balloon 405.

In some embodiments of the present invention, for the portion of stylet wire 250 that travels past pin vise 265, it may be desirable to increase the pushability of this section. Firstly, increasing the pushability may increase the tactile sensation by operator 70 during the extension of stylet 200 out from cutting cannula 210. This may also increase the amount of pushing force that can be applied by operator 70 without kinking of stylet wire 250 so that stylet 200 maybe extended out to its maximum travel during the stylet penetration step.

This may be enabled in a first embodiment by sliding a metal cannula over stylet wire 250 in this portion, where the metal cannula may be formed from nitinol or stainless steel, and may be stiffer than stylet wire 250. Furthermore, the metal cannula may be of sufficient length so that the metal cannula may function as a user interface (e.g., for operator access) for stylet wire 250. The inner diameter of the metal cannula may be sized to fit closely to that of stylet wire 250 so that both the metal cannula and stylet wire may be spot welded to one another. Similarly, the outer diameter of the metal cannula may be sized so as to permit passage through loosened pin vise 265, or pin vise 265 may be sized larger so as to accommodate a larger metal cannula.

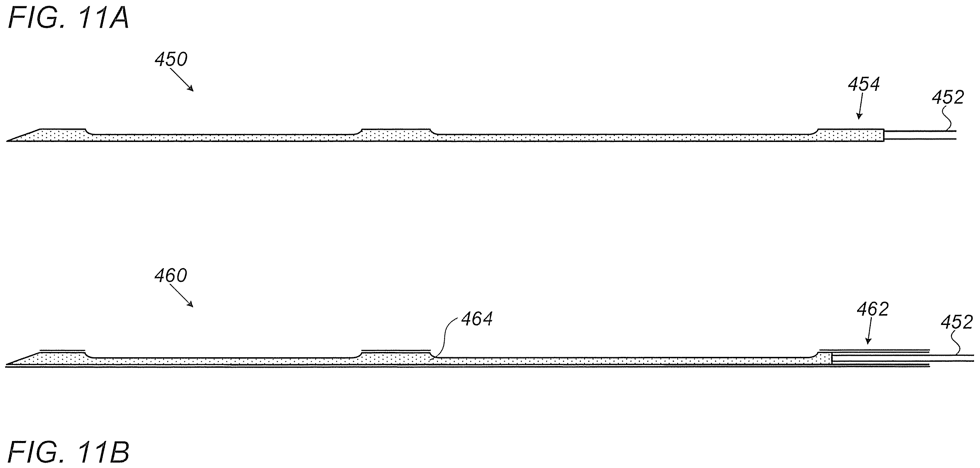

Fig. 11A schematically illustrates a solid stylet 450 joined end-to-end with a stylet wire 452, on accordance with some embodiments of the present invention.

Fig. 11B schematically illustrates a hollow stylet 460 overlappingly joined with stylet wire 452, on accordance with some embodiments of the present invention.

Stylet 450 as shown in Fig. 11A may be formed as single solid component, which means that the stylet - stylet wire joint may include an end-to-end joint 454, which may be technically challenging to fabrication. One way to overcome this is to have a hollow stylet design 464, such that an overlapping joint 462 may be formed, as shown in Figure 11B, while the outer profile remains substantially the same as solid stylet 450. The hollow regions of the stylet at overlapping joint 462 may be filled with medical grade adhesives so as increase adhesion between stylet wire 452 in overlapping joint 462.

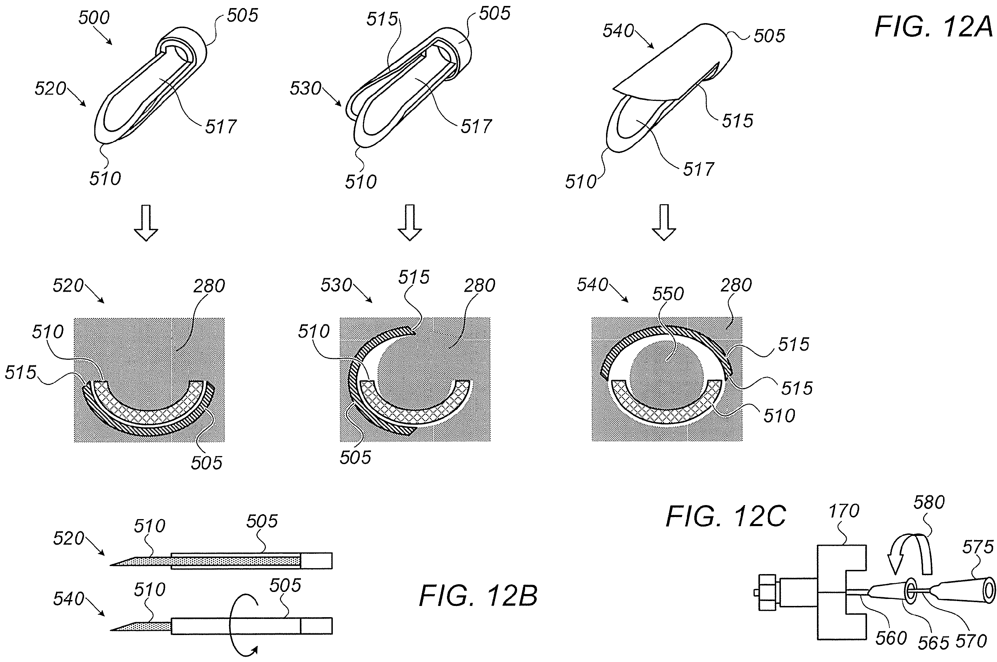

Fig. 12A schematically illustrates a torque-based cut biopsy needle 500 with an outer stylet 505 and an inner stylet 510, in accordance with some embodiments of the present invention.

Fig. 12B schematically illustrates a top view of torque-based cut biopsy needle 500 in an open configuration 520 and an encapsulation configuration 540, in accordance with some embodiments of the present invention.

Fig. 12C schematically illustrates rotating an outer stylet grip 565 for performing a torque-based cut needle biopsy, in accordance with some embodiments of the present invention.

Outer stylet 505 may include a knife cutting edge 515. Inner stylet 510 may include a specimen notch 517 as shown in Fig. 12A. In some embodiments, the width of outer stylet 505 may be 16G and the width of inner stylet 510 may be 18G.

Outer stylet 505 and inner stylet 510 may be respectively coupled to an outer stylet wire 560 and an inner stylet wire 570 as shown in Fig. 12C. The concentric inner 570 and outer 560 stylet wires may be connected at their proximal ends ex vivo to stylet grips for use by operator 70. For example, an outer stylet grip 565 may be attached to outer stylet wire 560, and an inner stylet grip 575 may be attached to inner stylet wire 570. Outer stylet 505 and inner stylet 510 may be threaded through guide catheter 105 in balloon-stabilized catheter body 100 such that the inner 510 and outer 505 stylets may be pushed into by operator 70 and/or fired by the handle into liver parenchyma 280.

In some embodiments of the present invention, torque-based cut biopsy needle 500 may be used to cut the soft tissue of liver parenchyma 280 using knife edge 515. Once the stylets are within liver parenchyma 280, operator 70 holding inner stylet grip 575 fixed may rotate outer stylet grip 565 ex vivo as shown in arrow 580 causing outer stylet 505 in vivo to rotate in liver parenchyma 280 relative to fixed inner stylet 510. As outer stylet 505 rotates, for example, cutting edge 515 cuts soft tissue from liver parenchyma 280 in a cutting configuration 530 from open configuration 520 and to encapsulation configuration 540 (e.g., rotation of 180 degrees) whereby a soft tissue sample 550 may be sheared off and encapsulated in specimen notch 517.

In other embodiments, the rotation of outer stylet 505 relative to inner stylet 510 may be enabled by any suitable rotating mechanism manual or automatic, for example, such as a computer-controlled rotational motor, for example. In some embodiments, a rotational torque of 0.2-0.22 Nm may be applied to outer stylet grip 565 ex vivo to shear the soft tissue in vivo using the torque-based biopsy mechanism described herein.

After soft tissue sample 550 is encapsulated in specimen notch 517, torque-based cut biopsy needle 500 may be withdrawn from the body of patient 65 (e.g., from guide catheter 105) while maintaining encapsulation configuration 540 so as to inspect encapsulated soft tissue biopsy sample 550.

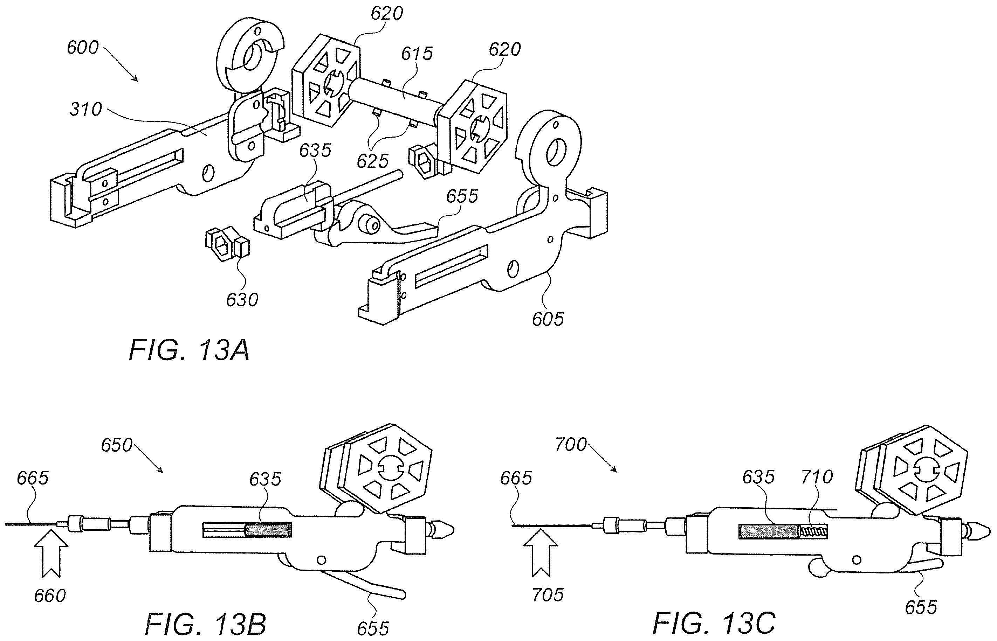

Fig. 13A schematically illustrates an exploded view of a second embodiment of a handle 600, in accordance with some embodiments of the present invention. Handle 600 may include a left handle portion 605, a right handle portion 610, a windlass axle 615, a windlass handle 620, windlass locking pins 625, a handle luer holder 630, an inner catheter bolt 635, and a firing lever 655.

Fig. 13B schematically illustrates handle 600 before firing 650, in accordance with some embodiments of the present invention. An arrow 660 illustrates a position of a stylet wire 665 and inner catheter bolt 635 in the cocked position.

Fig. 13C schematically illustrates handle 600 after firing 700, in accordance with some embodiments of the present invention. An arrow 705 illustrates the relative position of stylet wire 665 after firing as well as inner catheter bolt 635 revealing a spring 710. Handle 600 may be used to hold and lock inner catheter bolt 635 so that cutting cannula 210 will not fire until firing lever 655 is squeezed.

In some embodiments of the present invention, handles 255 and 600, for example, are typically configured to deliver an average maximum force applied by the stylet end of the cut biopsy needles to liver parenchyma 280 of about 1.7 N. Depending on the level of fibrosis in the liver tissue, the average force needed to penetrate liver parenchyma 280 may be in the range of 0.6-1.3 N. Hence, handles 255 and 600 shown herein deliver more force than is needed to penetrate liver parenchyma 280.

Portal hypertension may be caused by chronic liver diseases, which is characterized by an increased portal pressure gradient (PPG) as the difference in pressure between the portal vein and the inferior vena cava [IVC]. In normal conditions, the PPG may range between 1 and 5 mmHg. Portal hypertension becomes clinically significant when the PPG increases to 10 mmHg or above. Values between 5 and 9 mmHg represent subclinical portal hypertension. However, PPG may also be assessed by measuring the hepatic venous pressure gradient (HVPG), which is the difference between the wedged hepatic venous pressure (WHVP) and the free hepatic venous pressure (FHVP).

The WHVP may be measured by occluding the hepatic vein, e.g., stopping the blood flow cases the static column of blood, so as to equalize pressure in the preceding vascular territory, in this case the hepatic sinusoids. Thus, WHVP is a measure of hepatic sinusoidal pressure, not of portal pressure.

In a normal liver, WHVP is slightly lower (e.g., by about 1 mmHg) than portal pressure, owing to pressure equilibration through the interconnected sinusoids. In liver cirrhosis, however, the static column of blood created by occluding the hepatic vein cannot be decompressed at the sinusoidal level because the connection between sinusoids are disrupted as a result of the presence of fibrous septa and nodule formation. In cirrhosis, therefore, WHVP gives an accurate estimate of portal pressure, as has been demonstrated both for alcoholic and viral cirrhosis. FHVP is a measure of the pressure of the unoccluded hepatic vein.

HVPG is a measure of portal pressure, so the value changes, when the factors that determine portal pressure (e.g., resistance and blood flow) are modified. Changes in hepatic resistance may be caused by structural pathologies (fibrosis, regenerative nodules, or thrombosis) or functional abnormalities (increased hepatic vascular tone), or by changes in the portal or collateral blood.

In some embodiments of the present invention, in addition to the acquisition of a liver biopsy sample using balloon-stabilized catheter body 100 or 100B, the biopsy device itself may be coupled to a measurement system for assessing HVPG.

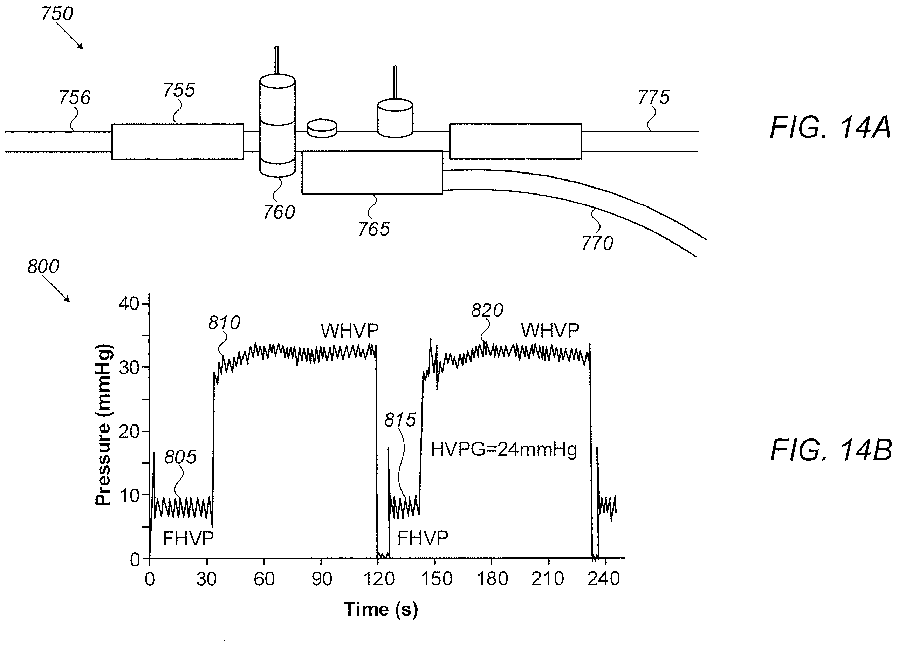

Fig. 14A schematically illustrates an adapter 750 for coupling balloon-stabilized catheter body 100 or 100B to a hepatic venous pressure gradient (HVPG) measurement system, in accordance with some embodiments of the present invention. Adapter 750 may include a luer lock 755 coupled to a catheter 756, a three-way tap 760, a pressure transducer 760, a tube 775 with pressured saline, and a cable 770 for coupling signals from pressure transducer 765 to a signal processor (not shown). Pressure transducer 760 may be used to measure HPVG from the pressure of blood in catheter 756. In some embodiments, luer lock 755 and catheter 756 shown in Fig. 14A may be, for example, luer lock 165 and guide catheter 105 of balloon-stabilized catheter body 100 as shown in Fig. 4A.

Catheterization of the hepatic vein may be carried out under sedation in conjunction with noninvasive vital sign monitoring (e.g., by electrocardiography, arterial blood pressure, and pulse oximetry). Under local anesthesia, the right jugular vein (or the femoral or antecubital vein) may be catheterized. A venous introducer (e.g., introducer sheath) may be placed into the vein. Balloon-stabilized catheter body 100 or 100B may be inserted through the venous introducer and navigated under fluoroscopic control into inferior vena cava 270 and hepatic vein 77, so as to measure WHVP, FHVP and pressure in the inferior vena cava with the balloon-stabilized catheter body that is coupled to an adapter 750 and HVPG measurement system.

Fig. 14B is a graph 800 of a hepatic venous pressure gradient (HVPG) measurement, in accordance with some embodiments of the present invention. FHVP 805 and 815 may be measured by maintaining distal tip 125 of the balloon-stabilized catheter body in the hepatic vein with balloons 120 or 120B deflated at about 2-4 cm, for example, from its opening into the inferior vena cava. The FHVP should be similar in value to the inferior vena cava pressure. A difference of more than 2 mmHg between FHVP and the inferior vena cava pressure may signify that the catheter may be inadequately placed, or that a hepatic vein obstruction may exist.

WHVP 810 and 820 may be measured by occluding the hepatic vein, either by wedging the catheter into a small branch of the hepatic vein or by inflating balloon 120 or 120B in balloon-stabilized catheter body 100 or 100B at distal tip 125. Adequate occlusion of the hepatic vein may be confirmed by slowly injecting 5 ml of a contrast dye into the vein (e.g., a procedure that reveals a typical 'wedged' pattern) without observing reflux of the dye or washout through communications with other hepatic veins. However, occlusion of the hepatic vein using balloon inflation may include a larger volume of liver circulation that is detected relative to wedging the catheter. Balloon inflation HVPG may exhibit better measurement sensitivity or variability. WHVP 810 and 820 are measured until the value stabilizes usually after about 40 s, for example.

A hepatic venous pressure gradient (HVPG) measurement system may include adapter 750, a recorder, and a signal processing unit where cable 770 may couple the signal from pressure transducer 765 to the signal processing unit. All measurement are taken at least in duplicate as shown in graph 800. Permanent tracings (e.g., graph 800) may be obtained with a multichannel recorder and adequately calibrated transducers (e.g., pressure transducer 765). The example HVPG measurement shown in graph 800 of Fig. 14B was made with a recorder speed of 1 mm/sec. The data was acquired in about 25 seconds. The signal processing unit may compute HVPG as is the difference between the measured wedged hepatic venous pressure (WHVP) and the measured free hepatic venous pressure (FHVP).

In some embodiment of the present invention, measuring FHVP may be performed by inserting the balloon-stabilized catheter body into a guidewire, removing the guidewire, connecting the balloon-stabilized catheter body to the pressure transducer, injecting contrast through lumen of the first elongated tube to check position, and plotting pressure on the recorder. Measuring WHVP may be performed by inflating balloon 120, injecting contrast to verify that that the catheter was successfully wedged, and plotting pressure on the recorder. The guidewire may be reinsert and the biopsy procedure started using the balloon-stabilized catheter body.

In some embodiments of the present invention, a balloon-anchored, biopsy device 90 for acquiring a biopsy sample of a target organ in a subject may include a first elongated tube, a second elongated tube, and a flexible biopsy needle. The first elongated tube (e.g., balloon catheter 110) may enclose a first lumen with a first proximal end and a distal tip, where a section of the first elongated tube near the distal tip may include a balloon that when inserted into a blood vessel of a target organ of a subject and inflated, anchors the section in the blood vessel near a biopsy site in the target organ.

The second elongated tube (e.g., guide catheter 105) may enclose a second lumen with a second proximal end and a second distal end comprising a beveled distal exit of the second lumen, which is positioned at the biopsy site of the target organ when the first elongated tube is anchored in the blood vessel by the inflated balloon, where a predefined length of the first and the second elongated tubes are longitudinally attached to one another such that the beveled distal exit is positioned at a proximal end of the section of the first elongated tube.

The flexible biopsy needle may be attached to a distal end of a wire for insertion into the second lumen of the second elongated tube for navigation to the biopsy site, wherein the flexible biopsy needle is configured to exit the beveled distal exit of the second lumen for penetration into tissue of the target organ at the biopsy site at a predefined angle between a longitudinal axis of the section of the first elongated tube and a longitudinal axis of the flexible biopsy needle, and to acquire a biopsy sample of the target organ at the biopsy site.

In some embodiments of the present invention, the first and the second elongated tubes may be formed from extruded adjacent tubes. They may include multiple lumens, or any combination thereof.

In some embodiments of the present invention, the first elongated tube may include a multi-lumen tube with a 2-lumen configuration: one lumen for guide wire and the other lumen to inflate balloon at the distal tip. The second elongated tube may be a single lumen. Both first and second elongated tubes may be formed by extrusion one lumen adjacent to the other. Additionally and/or optionally, the second elongated tube may be coextruded.

In some embodiments of the present invention, the second elongated tube may include a tougher inner layer so as to prevent the flexible biopsy needle from penetrating or damaging (e.g., such that debris is generated) the walls when navigating through bends from the peripheral veins to the hepatic veins. The tough inner layer may also include a metal coil.

In some embodiments of the present invention, the first and second elongated tubes may be held together using a heat shrink tube.

In some embodiments of the present invention, the second elongation tube may include a rigid contoured section 131 that may rest on the balloon such that when the balloon is inflated, the balloon may lift rigid contoured section 131 of the second elongation tube. This effect increases angle ϕ as shown in Fig. 7D so as to better allow the biopsy needle to face the wall of the hepatic vein when fired into the liver tissue. This effect may also increase the likelihood of a successful biopsy, since the biopsy needle is more likely to exit hepatic vein and enter into the liver tissue. Unlike the transjugular approach, there is no stiff metal tubing to permit rotation such that biopsy needle will be directed into the liver tissue. The effect may also prevent the biopsy needle piercing the balloon directly front of it.

In some embodiments of the present invention, the flexible biopsy needle may include a Tru-cut biopsy needle.

In some embodiments of the present invention, the first elongated tube and the second elongated tube may respectively include a balloon catheter and a guide catheter.

In some embodiments of the present invention, the target organ may include a liver.

In some embodiments of the present invention, the blood vessel may include a hepatic vein of the liver.

In some embodiments of the present invention, the biopsy device may include a locking mechanism coupled to the second proximal end for fixing the position of the flexible biopsy needle at the distal end of the wire in the second lumen.

In some embodiments of the present invention, components of the locking mechanism are selected from the group consisting of a Tuohy Borst adapter, a luer lock, and a compressible clamp.

In some embodiments of the present invention, the flexible biopsy needle may include a cutting cannula and a stylet.

In some embodiments of the present invention, the flexible biopsy needle may include flattened band and an alignment notch for maintaining an alignment of the cutting cannula and the stylet.

In some embodiments of the present invention, the first elongated tube is more flexible the second elongated tube. The term "more flexible" used in the context herein means that the first elongated tube (e.g., balloon catheter 110) may be more pliant than the second elongated tube (e.g., guide catheter 105). The first elongated tube may be capable of being flexed and/or bent more than the second elongated tube. In some embodiments, the first elongated tube may be formed from a first material more pliant than a second material forming the second elongated tube. In other embodiments, the geometries of the first elongated tube and geometries of the second elongated tube may cause the first elongated tube to be more pliant or bendable relative to the second elongated tube.

In some embodiments of the present invention, the biopsy device may include an outer tube with the predefined length into which the first elongated tube and the second elongated tube are inserted so as to longitudinally attach the first elongated tube and the second elongated tube to one another.

In some embodiments of the present invention, the diameter of the inflated balloon may be larger than the diameter of the blood vessel.

In some embodiments of the present invention, the diameter of the inflated balloon may be no larger than 20% of the diameter of the blood vessel.

In some embodiments of the present invention, the predefined angle is in the range of 15-45 degrees.

In some embodiments of the present invention, the biopsy device may include a connecting tube for insertion into the second lumen for guiding the flexible biopsy needle at the distal end of the wire to the biopsy site.

In some embodiments of the present invention, the flexible biopsy needle may include a stylet joined to a stylet wire in an end-to-end joint.

In some embodiments of the present invention, the flexible biopsy needle may include a hollow stylet and a stylet wire inserted into an overlapping joint.

In some embodiments of the present invention, the flexible biopsy needle may include an inner stylet and outer stylet with a cutting edge arranged in a concentric configuration.

In some embodiments of the present invention, the flexible biopsy needle may be configured to acquire the biopsy sample by rotating the outer stylet with the cutting edge relative to the inner stylet when the flexible biopsy needle is within the tissue of the target organ.

In some embodiments of the present invention, the flexible biopsy needle may be configured to encapsulate the acquired biopsy sample in a specimen notch when the outer stylet remains in a rotated position substantially opposite to the inner stylet.

In some embodiments of the present invention, the biopsy device may include a rigid contoured section coupled to the distal end of the second elongated tube for increasing the predefined angle when the balloon is inflated.

In some embodiments of the present invention, the balloon may include a distal balloon and a proximal balloon, which are inflatable separately or together in the blood vessel.

In some embodiments of the present invention, the second elongated tube may be coupled to a pressure transducer for measuring a hepatic venous pressure gradient (HVPG) by processing a signal from the pressure transducer in a signal processing unit.

In some non-claimed examples, a method for acquiring a biopsy sample of a target organ of a subject using a balloon-anchored biopsy device may include percutaneously inserting a biopsy device into a vein of a limb of a subject. The biopsy device may include a first elongated tube, a second elongated tube, and a flexible biopsy needle.

The first elongated tube may enclose a first lumen with a first proximal end and a distal tip, wherein a section of the first elongated tube near the distal tip may include a balloon that when inserted into a blood vessel of a target organ of a subject and inflated, anchors the section in the blood vessel near a biopsy site in the target organ.

The second elongated tube may enclose a second lumen with a second proximal end and a second distal end may include a beveled distal exit of the second lumen, which is positioned at the biopsy site of the target organ when the first elongated tube is anchored in the blood vessel by the inflated balloon, wherein a predefined length of the first and the second elongated tubes are longitudinally attached to one another such that the beveled distal exit is positioned at a proximal end of the section of the first elongated tube.

The flexible biopsy needle may be attached to a distal end of a wire for insertion into the second lumen of the second elongated tube for navigation to the biopsy site, wherein the flexible biopsy needle is configured to exit the beveled distal exit of the second lumen for penetration into tissue of the target organ at the biopsy site at a predefined angle between a longitudinal axis of the section of the first elongated tube and a longitudinal axis of the flexible biopsy needle.

The method for acquiring the biopsy sample may further include the distal tip being navigated from the vein through a vascular system of the subject and into the blood vessel of the target organ near the biopsy site. The balloon may be inflated in the blood vessel. The flexible biopsy needle may be pushed into the tissue of the target organ at the biopsy site at the predefined angle. A biopsy sample of the target organ at the biopsy site may be acquired using the flexible biopsy needle. The wire may be withdrawn from the second lumen so as to retrieve the acquired biopsy sample.

In some examples, the limb may include an arm of the subject and the vein may include a cephalic vein of the arm.

In some examples, the limb may include a leg of the subject and the vein may include a femoral vein of the leg.

In some examples, percutaneously inserting the biopsy device into the vein of the limb of the subject may include inserting the biopsy device through a lumen of a sheath in the vein.

In some examples, the balloon may include a distal balloon and a proximal balloon, and the method may include inflating the balloon comprises inflating the distal balloon and the proximal balloon separately or together in the blood vessel.

In some examples, the second elongated tube may be coupled to a pressure transducer, and the method may include measuring a hepatic venous pressure gradient (HVPG) by processing a signal from the pressure transducer.

Different embodiments are disclosed herein. Features of certain embodiments may be combined with features of other embodiments; thus certain embodiments may be combinations of features of multiple embodiments. The foregoing description of the embodiments of the invention has been presented for the purposes of illustration and description. It is not intended to be exhaustive or to limit the invention to the precise form disclosed. It should be appreciated by persons skilled in the art that many modifications, variations, substitutions, changes, and equivalents are possible in light of the above teachings, provided that they fall within the scope of the invention which is defined by the appended claims.

## Claims

1. A balloon-anchored, biopsy device (90) for acquiring a biopsy sample of a target organ in a subject (65), the biopsy device comprising:
a first elongated tube enclosing a first lumen (109) with a first proximal end and a distal tip, wherein a section of the first elongated tube near the distal tip comprises a balloon (120) that when inserted into a blood vessel of a target organ of a subject and inflated, anchors the section in the blood vessel near a biopsy site in the target organ;
a second elongated tube enclosing a second lumen (106) with a second proximal end and a second distal end comprising a beveled distal exit (130) of the second lumen, which is positioned at the biopsy site of the target organ when the first elongated tube is anchored in the blood vessel by the balloon which is inflated, wherein a predefined length of the first and the second elongated tubes are longitudinally attached to one another such that the beveled distal exit (130) is positioned at a proximal end of the section of the first elongated tube; and
a flexible biopsy needle (190) attached to a distal end of a wire for insertion into the second lumen of the second elongated tube for navigation to the biopsy site, wherein the flexible biopsy needle (190) is configured to exit the beveled distal exit (130) of the second lumen for penetration into tissue of the target organ at the biopsy site at a predefined angle between longitudinal axis of the section of the first elongated tube and longitudinal axis of the flexible biopsy needle (190), and to acquire a biopsy sample of the target organ at the biopsy site; and
**characterized in that** the first elongated tube is more flexible than the second elongated tube.

2. The biopsy device (90) according to claim 1, wherein the flexible biopsy needle (190) comprises a Tru-cut biopsy needle, the target organ comprises a liver (75) and the blood vessel comprises a hepatic vein (77) of a liver of the subject, and wherein the first elongated tube and the second elongated tube respectively are a balloon catheter (110) and a guide catheter (105).

3. The biopsy device (90) according to claim 1 or 2, further comprising a locking mechanism coupled to the second proximal end for fixing the position of the flexible biopsy (190) needle at the distal end of the wire in the second lumen.

4. The biopsy device (90) according to claim 2, wherein the Tru-cut biopsy needle is a distally self-aligned flexible Tru-cut needle.

5. The biopsy device (90) according to claim 1, wherein the flexible biopsy needle (190) comprises a cutting cannula (210) and a hollow stylet (460), a flattened band (225) and an alignment notch (235) for maintaining an alignment of the cutting cannula and the hollow stylet, the hollow stylet is joined to a stylet wire in an end-to-end joint, the stylet wire inserted into an overlapping joint.

6. The biopsy device (90) according to claim 3, wherein the components of the locking mechanism are selected from the group consisting of a Tuohy Borst adapter, a luer lock, and a compressible clamp.

7. The biopsy device according to claim 1, further comprising an outer tube with the predefined length into which the first elongated tube and the second elongated tube are inserted so as to longitudinally attach the first elongated tube and the second elongated tube to one another.

8. The biopsy device according to claim 1, wherein the diameter of the balloon which is inflated is larger than the diameter of the blood vessel, and wherein the predefined angle is in a range of 15-45 degrees.

9. The biopsy device according to claim 1, further comprising a connecting tube for insertion into the second lumen for guiding the flexible biopsy needle (190) at the distal end of the wire to the biopsy site.

10. The biopsy device according to claim 1, wherein the flexible biopsy needle (190) comprises a stylet joined to a stylet wire in an end-to-end joint.

11. The biopsy device according to claim 1, wherein the flexible biopsy needle (190) comprises an inner stylet and outer stylet with a cutting edge arranged in a concentric configuration.

12. The biopsy device according to claim 11, wherein the flexible biopsy needle (190) is configured to acquire the biopsy sample by rotating the outer stylet with the cutting edge relative to the inner stylet when the flexible biopsy needle (190) is within the tissue of the target organ.

13. The biopsy device according to claim 1, further comprising a rigid contoured section coupled to the distal end of the second elongated tube for increasing the predefined angle when the balloon is inflated.

14. The biopsy device according to claim 1, wherein the balloon comprises a distal balloon and a proximal balloon, which are inflatable separately or together in the blood vessel.

15. The biopsy device according to claim 1, wherein the second elongated tube is coupled to a pressure transducer for measuring a hepatic venous pressure gradient (HVPG) by processing a signal from the pressure transducer in a signal processing unit.

## Patentansprüche

1. Ballonverankerte Biopsievorrichtung (90) zur Gewinnung einer Biopsieprobe eines Zielorgans in einem Subjekt (65), wobei die Biopsievorrichtung umfasst:
eine erste längliche Röhre, die ein erstes Lumen (109) mit einem ersten proximalen Ende und einer distalen Spitze umschließt, wobei ein Abschnitt der ersten länglichen Röhre in der Nähe der distalen Spitze einen Ballon (120) umfasst, der, wenn er in ein Blutgefäß eines Zielorgans eines Patienten eingeführt und aufgeblasen wird, den Abschnitt in dem Blutgefäß in der Nähe einer Biopsiestelle in dem Zielorgan verankert;
eine zweite längliche Röhre, die ein zweites Lumen (106) mit einem zweiten proximalen Ende und einem zweiten distalen Ende umschließt, das einen abgeschrägten distalen Ausgang (130) des zweiten Lumens umfasst, der an der Biopsiestelle des Zielorgans positioniert wird, wenn die erste längliche Röhre durch den aufgeblasenen Ballon in dem Blutgefäß verankert wird, wobei eine vordefinierte Länge der ersten und der zweiten länglichen Röhre in Längsrichtung aneinander befestigt sind, so dass der abgeschrägte distale Ausgang (130) an einem proximalen Ende des Abschnitts des ersten länglichen Schlauchs positioniert ist; und
eine flexible Biopsienadel (190), die an einem distalen Ende eines Drahtes zum Einführen in das zweite Lumen der zweiten länglichen Röhre zur Navigation zur Biopsiestelle befestigt ist, wobei die flexible Biopsienadel (190) so konfiguriert ist, dass sie aus dem abgeschrägten distalen Ausgang (130) des zweiten Lumens austritt, um in das Gewebe des Zielorgans an der Biopsiestelle in einem vordefinierten Winkel zwischen der Längsachse des Abschnitts des ersten länglichen Röhre und der Längsachse der flexiblen Biopsienadel (190) einzudringen und eine Biopsieprobe des Zielorgans an der Biopsiestelle zu gewinnen;
und **dadurch gekennzeichnet ist, dass** die erste längliche Röhre flexibler ist als die zweite längliche Röhre.

2. Biopsievorrichtung (90) nach Anspruch 1, wobei die flexible Biopsienadel (190) eine Tru-Cut-Biopsienadel umfasst, das Zielorgan eine Leber (75) umfasst und das Blutgefäß eine Lebervene (77) einer Leber des Subjekts umfasst, und wobei die erste längliche Röhre und die zweite längliche Röhre jeweils ein Ballonkatheter (110) und ein Führungskatheter (105) sind.

3. Biopsievorrichtung (90) nach Anspruch 1 oder 2, die ferner einen Verriegelungsmechanismus umfasst, der mit dem zweiten proximalen Ende gekoppelt ist, um die Position der flexiblen Biopsienadel (190) am distalen Ende des Drahtes in dem zweiten Lumen zu fixieren.

4. Biopsievorrichtung (90) nach Anspruch 2, wobei die Tru-Cut-Biopsienadel eine distal selbstausrichtende flexible Tru-Cut-Nadel ist.

5. Biopsievorrichtung (90) nach Anspruch 1, wobei die flexible Biopsienadel (190) eine Schneidekanüle (210) und ein hohles Stilett (460), ein abgeflachtes Band (225) und eine Ausrichtungskerbe (235) zum Aufrechterhalten einer Ausrichtung der Schneidekanüle und des hohlen Stilettes umfasst, wobei das hohle Stilett mit einem Stilettdraht in einer End-zu-End-Verbindung verbunden ist, wobei der Stilettdraht in eine überlappende Verbindung eingeführt ist.

6. Biopsievorrichtung (90) nach Anspruch 3, wobei die Komponenten des Verriegelungsmechanismus ausgewählt sind aus der Gruppe bestehend aus einem Tuohy-Borst-Adapter, einem Luer-Lock und einer komprimierbaren Klemme.

7. Biopsievorrichtung nach Anspruch 1, die ferner eine äußere Röhre mit der vordefinierten Länge umfasst, in die die erste längliche Röhre und die zweite längliche Röhre eingeführt werden, um die erste längliche Röhre und die zweite längliche Röhre in Längsrichtung aneinander zu befestigen.

8. Biopsievorrichtung nach Anspruch 1, wobei der Durchmesser des aufgeblasenen Ballons größer ist als der Durchmesser des Blutgefäßes und wobei der vordefinierte Winkel in einem Bereich von 15-45 Grad liegt.

9. Biopsievorrichtung nach Anspruch 1, ferner umfassend eine Verbindungsröhre zum Einführen in das zweite Lumen, um die flexible Biopsienadel (190) am distalen Ende des Drahtes zur Biopsiestelle zu führen.

10. Biopsievorrichtung nach Anspruch 1, wobei die flexible Biopsienadel (190) ein Stilett umfasst, das mit einem Stilettdraht in einer End-zu-End-Verbindung verbunden ist.

11. Biopsievorrichtung nach Anspruch 1, wobei die flexible Biopsienadel (190) ein inneres Stilett und ein äußeres Stilett mit einer konzentrisch angeordneten Schneidkante umfasst.

12. Biopsievorrichtung nach Anspruch 11, wobei die flexible Biopsienadel (190) so konfiguriert ist, dass sie die Biopsieprobe durch Drehen des äußeren Stiletts mit der Schneidekante relativ zum inneren Stilett gewinnt, wenn sich die flexible Biopsienadel (190) im Gewebe des Zielorgans befindet.

13. Biopsievorrichtung nach Anspruch 1 umfasst ferner einen starren konturierten Abschnitt, der mit dem distalen Ende der zweiten länglichen Röhre verbunden ist, um den vordefinierten Winkel zu vergrößern, wenn der Ballon aufgeblasen wird.

14. Biopsievorrichtung nach Anspruch 1, wobei der Ballon einen distalen Ballon und einen proximalen Ballon umfasst, die separat oder zusammen in dem Blutgefäß aufblasbar sind.

15. Biopsievorrichtung nach Anspruch 1, wobei die zweite längliche Röhre mit einem Druckwandler zur Messung eines hepatischen Venendruckgradienten (HVPG) durch Verarbeitung eines Signals von dem Druckwandler in einer Signalverarbeitungseinheit gekoppelt ist.

## Revendications

1. Dispositif de biopsie à ancrage par ballonnet pour l'acquisition d'un échantillon de biopsie d'un organe cible chez un sujet (65), le dispositif de biopsie comprenant :
un premier tube allongé renfermant une première lumière (109) avec une première extrémité proximale et une pointe distale, dans lequel une section du premier tube allongé près de la pointe distale comprend un ballon (120) qui, lorsqu'il est inséré dans un vaisseau sanguin d'un organe cible d'un sujet et gonflé, ancre la section dans le vaisseau sanguin près d'un site de biopsie dans l'organe cible ;
un second tube allongé renfermant une seconde lumière (106) avec une seconde extrémité proximale et une seconde extrémité distale comprenant une sortie distale (130) biseautée de la seconde lumière, qui est positionnée au site de biopsie de l'organe cible lorsque le premier tube allongé est ancré dans le vaisseau sanguin par le ballon gonflé, dans lequel une longueur prédéfinie des premier et second tubes allongés sont attachés longitudinalement l'un à l'autre de telle sorte que la sortie distale biseautée est positionnée à une extrémité proximale de la section du premier tube allongé ; et
une aiguille de biopsie flexible (190) fixée à une extrémité distale d'un fil pour insertion dans la seconde lumière du second tube allongé pour la navigation vers le site de biopsie, l'aiguille de biopsie flexible étant configurée pour sortir de la sortie distale (130) biseautée de la seconde lumière pour pénétrer dans le tissu de l'organe cible au niveau du site de biopsie à un angle prédéfini entre un axe longitudinal de la section du premier tube allongé et un axe longitudinal de l'aiguille de biopsie flexible (190), et pour acquérir un échantillon de biopsie de l'organe cible au niveau du site de biopsie et
**caractérisé en ce que** le premier tube allongé est plus flexible que le second tube allongé.

2. Dispositif de biopsie (90) selon la revendication 1, dans lequel l'aiguille de biopsie flexible comprend une aiguille de biopsie Tru-cut, l'organe cible comprend un foie (75) et le vaisseau sanguin comprend une veine hépatique (77) du foie du sujet, et dans lequel le premier tube allongé et le second tube allongé sont respectivement un cathéter à ballonnet (110) et un cathéter guide (105).

3. Dispositif de biopsie (90) selon la revendication 1 ou 2, comprenant en outre un mécanisme de verrouillage couplé à la seconde extrémité proximale pour fixer la position de l'aiguille de biopsie flexible (190) à l'extrémité distale du fil dans la seconde lumière.

4. Dispositif de biopsie (90) selon la revendication 2, dans lequel l'aiguille de biopsie Tru-cut est une aiguille Tru-cut flexible à alignement distal automatique.

5. Dispositif de biopsie (90) selon la revendication 1, dans lequel l'aiguille de biopsie flexible (190) comprend une canule de coupe (210) et un stylet creux (460), une bande aplatie (225) et une encoche d'alignement (235) pour maintenir l'alignement de la canule de coupe et du stylet creux, le stylet creux est relié à une broche de stylet dans une articulation bout à bout, la broche de stylet étant insérée dans une articulation chevauchante.

6. Dispositif de biopsie (90) selon la revendication 3, dans lequel les composants du mécanisme de verrouillage sont choisis dans le groupe constitué d'un adaptateur Tuohy Borst, d'un verrou Luer et d'une pince compressible.

7. Le dispositif de biopsie selon la revendication 1, comprenant en outre un tube extérieur de longueur prédéfinie dans lequel le premier tube allongé et le second tube allongé sont insérés de manière à fixer longitudinalement le premier tube allongé et le second tube allongé l'un à l'autre.

8. Dispositif de biopsie selon la revendication 1, dans lequel le diamètre du ballon gonflé est supérieur au diamètre du vaisseau sanguin, et dans lequel l'angle prédéfini est compris entre 15 et 45 degrés.

9. Dispositif de biopsie selon la revendication 1 comprend en outre un tube de connexion à insérer dans la seconde lumière pour guider l'aiguille de biopsie flexible (190) à l'extrémité distale du fil jusqu'au site de biopsie.

10. Dispositif de biopsie selon la revendication 1, dans lequel l'aiguille de biopsie flexible (190) comprend un stylet relié à un fil de stylet dans une articulation bout à bout.

11. Dispositif de biopsie selon la revendication 1, dans lequel l'aiguille de biopsie flexible (190) comprend un stylet interne et un stylet externe avec un bord coupant disposés dans une configuration concentrique.

12. Le dispositif de biopsie selon la revendication 11, dans lequel l'aiguille de biopsie flexible (190) est configurée pour acquérir l'échantillon de biopsie en faisant tourner le stylet externe avec le bord tranchant par rapport au stylet interne lorsque l'aiguille de biopsie flexible (190) se trouve dans le tissu de l'organe cible.

13. Le dispositif de biopsie selon la revendication 1, comprenant en outre une section rigide profilée couplée à l'extrémité distale du second tube allongé pour augmenter l'angle prédéfini lorsque le ballonnet est gonflé.

14. Dispositif de biopsie selon la revendication 1, dans lequel le ballonnet comprend un ballonnet distal et un ballonnet proximal, qui peuvent être gonflés séparément ou ensemble dans le vaisseau sanguin.

15. Dispositif de biopsie selon la revendication 1, dans lequel le second tube allongé est couplé à un transducteur de pression pour mesurer un gradient de pression veineuse hépatique (HVPG) en traitant un signal provenant du transducteur de pression dans une unité de traitement des signaux.
